# EUROPEAN PATENT SPECIFICATION

(11) **EP 3 122 330 B1**
(45) Date of publication and mention of the grant of the patent: **25.07.2018**
(21) Application number: 15712898.4
(22) Date of filing: 26.03.2015
(51) Int. Cl.: A61K 9/00, A61K 9/50, A61K 31/495, A61K 9/16

(54) **PHARMACEUTICAL COMPOSITIONS COMPRISING LEVOCETIRIZINE**
PHARMAZEUTISCHE ZUSAMMENSETZUNGEN BEINHALTEND LEVOCETIRIZIN
COMPOSITIONS PHARMACEUTIQUES COMPRENANT DE LA LÉVOCÉTIRIZINE

(30) Priority: 27.03.2014 EP 14162001; 27.03.2014 EP 14161997
(43) Date of publication of application: 01.02.2017
(73) Proprietor: UCB FARCHIM, S.A., 1630 Bulle (CH)
(72) Inventor: FANARA, Domenico, B-1070 Brussels (BE); GOOLE, Jonathan, B-1200 Brussels (BE); DELEERS, Michel, B-1650 Linkebeek (BE)
(74) Representative: UCB Intellectual Property
(86) International application number: PCT/EP2015/056593
(87) International publication number: WO 2015/144830

(56) References cited:
- WO-A2-2011/110939
- JP-A- 2009 114 069
- US-A1- 2002 032 217
- US-A1- 2006 083 786
- US-A1- 2012 010 218

## Description

The present invention relates to a solid oral pharmaceutical composition comprising levocetirizine as active ingredient as well as their pharmaceutically acceptable salts and mixtures thereof.

This active ingredient is orally active and selective histamine H₁-receptor antagonists. It is described in EP 0 058 146. Example of this compound includes levocetirizine, the (S) enantiomer of cetirizine, in its dihydrochloride form marketed under the tradename Xyzal®.

International patent application WO 03/059328 describes a dry syrup composition made of a mixture of two parts prepared in the form of granules, one part comprises, as active ingredient, cetirizine or levocetirizine and, as excipients, β cyclodextrin, acesulfam K, lactose monohydrate and sodium citrate (as an alcalinizing agent) and one other part comprises mannitol and flavor. The first was compacted (dry granulation), milled and sieved so that two types of granules were obtained, one comprising cetirizine or levocetirizine and one comprising mannitol, as a polyol. The final composition of the dry syrups were obtained by mixing the two types of granules.

Moreover, application WO 03/059328 explains that polyols can react with levocetirizine or cetirizine and that they could be classified as very reactive polyols (*viz.* polyols with molecular weight below 300 g/mol) or reactive polyols (*viz.* polyols with molecular weight between 300 and 950, with exception to lactose). In order to control the degradation, the molar ratio between the very reactive polyol in intimate contact with the active ingredient and the active ingredient should not be higher than 10, preferably 5. Therefore, the formulation had to be split in two parts, to avoid intimate contact between very reactive polyols that are present in excess higher than 10 and the active ingredient.

US patent application US 2002/032217 describes a cetirizine composition containing cyclodextrine as taste masking. Application WO 2011/110939 discloses taste-masked, immediate release compositions comprising levocetirizine that remain stable in the presence of polyols. The aim of the present invention is to provide oral pharmaceutical compositions, in a solid form, and aiming to be used for children aged from 6 months and for adults. As dosage variability should be avoided in the packaging, the concentration of active ingredient in the formulation needs to be reduced below the concentration exampled in WO 03/059328, in order to be able to manufacture the lowest doses and have enough fill weight. Despite this low concentration, the composition based on a granular form has to be stable and homogeneous.

Moreover, the problem to be solved by the present invention was also to obtain oral compositions having acceptable taste and palatability when dispersed in water or swallowed directly, whilst maintaining optimal immediate release kinetics for the active ingredient.

Moreover, it is also the aim of the present invention to provide a solid unit dose pharmaceutical composition that makes a clear solution, or alternatively an homogeneous translucid suspension, when dispersed in a glass of water.

One of the objectives of the invention is a pharmaceutical composition which can be administered orally to obtain an immediate release of pharmaceutically active ingredients. The term of "Immediate Release" composition is understood here as a composition having an *in-vitro* dissolution release as described in FDA guidance (Guidance for industry dissolution testing for oral dosage form FDA 1997). Usually, the composition has an *in-vitro* dissolution release of at least 80% in 30 min; and preferably of at least 85% in 15 min. while using USP2 dissolution method.

It has now surprisingly been found that the above problems can be solved by using a formulation containing a single type of active granule produced as part of the manufacturing process of the pharmaceutical composition despite the presence of a low molecular weight polyol. This can only be achieved by buffering an aqueous solution of the active ingredient during the preparation process of the active granule. In this way, a homogeneous dispersion of the active ingredient is obtained while assuring a good stability of the active ingredient in the granule and in the final pharmaceutical composition.

The present invention provides pharmaceutical compositions, in a solid form, allowing an oral administration of an unit dose ranging from 0.50 mg to 25.00 mg of levocetirizine dihydrochloride, as active ingredient, and containing active granules comprising
- the active ingredient,
- a polyol fraction comprising one or more solid water soluble polyols having a molecular weight below 950 g/ mol, and with a molar ratio between the polyol fraction and the active ingredient higher than 50, at least one solid water soluble polyol being mannitol, and
- a buffering system which contributes to maintain the pH of the whole pharmaceutical composition between 4.0 and 7.0 when the pharmaceutical composition containing 5 mg of active ingredient is dissolved in 100 ml of water.

Usually, the active granule produced as part of the manufacturing process of the pharmaceutical composition comprises only one active ingredient, it does not comprise any other drug.

Usually, the pharmaceutical compositions, containing the active granules, comprise only one active ingredient, it does not comprise any other drug.

Levocetirizine dihydrochloride is the sole active pharmaceutical ingredient in the composition.

Preferably, the composition contain one type of active granules. Preferably, all the active granules contained in the composition comprise an active ingredient.

Preferably, the pharmaceutical composition of the invention allows an oral administration of an unit dose ranging from 1.00 to 10.00 mg of active ingredient.

All % are here expressed in weight %, except specified otherwise.

The term " water soluble " polyol means a polyol at least sparingly soluble in water according to the European Pharmacopeia 7^{th} edition definition, at room temperature under atmospheric pressure.

A solid polyol is defined as a polyol which is not liquid at room temperature under atmospheric pressure.

The term "Unit dose" means an amount of the pharmaceutical composition suitable for the administration of the required dose of active ingredient while being suitable for patient compliance and for processing.

The term " active granules" is here understood as individual particles that contain the active ingredient. Active granules have been obtained by a pharmaceutically accepted process and that consist in small particles of different or same nature gathered into a larger one, or in individual particles build from ingredients from different nature or coated by different ingredients. Individual solid starting materials entering into the formulation of the pharmaceutical composition are not considered as active granules.

Usually the mean particle size of the active granules produced as part of the manufacturing process of the pharmaceutical composition is comprised between 50 µm and 1000 µm. Preferably, the mean particle size of these active granules is comprised between 100 µm and 800 µm. Best results have been obtained with active granules having mean particle size ranging from 150 to 600 µm.

Usually, the pharmaceutical composition according to the present invention comprises 0.1 to 4.0 % per weight of active compound with respect to the total weight of the composition . Particularly, the pharmaceutical composition according to the present invention comprises 0.1 to 2.0 % per weight of active compound with respect to the total weight of the composition. Preferably, the pharmaceutical composition according to the present invention comprises 0.1 to 1.5 % per weight of active compound with respect to the total weight of the composition. More preferably, the pharmaceutical composition according to the present invention comprises 0.1 to 1.0 % per weight of active compound with respect to the total weight of the composition.

In a specific embodiment, the pharmaceutical composition comprises active granules in an amount of 25 to 100 %, preferably 50 to 100 % with respect to the total weight of the pharmaceutical composition; if the content of active granules in the pharmaceutical composition is lower than 100 % , then the composition is completed with extragranular excipients.

Usually, the active granule comprises a polyol fraction with a molar ratio between the polyol and the active ingredient higher than 50, preferably higher than 75 and more preferably higher than 100.

In a specific embodiment, when the pharmaceutical composition is a dry syrup composition, the active granule comprises a polyol fraction with a molar ratio between the polyol and the active ingredient higher than 100, preferably higher than 150 and more preferably higher than 200.

Usually, the active granule comprises at least a solid water soluble polyol having a molecular weight below 950 g/ mol, preferably below 350 g/mol.

Examples of polyols are sorbitol, xylitol, maltitol, dextrose, sucrose, mannitol, maltose, isomalt, lactose and mixture thereof. Most preferably, the polyol fraction does not include lactose.

Preferably, the polyols are maltitol, dextrose, mannitol, isomalt, and mixture thereof. Best results have been obtained with mannitol, the claims disclose mannitol. The active granule contains at least 50% of polyol fraction, usually at least 60 % of polyol fraction and preferably at least 70% of polyol fraction with respect of the total weight of the granules.

Lactose can be used as filler in the pharmaceutical compositon or in the active granule, but it is not considered to be part of the polyol fraction.

The active granule comprises a buffering system which contributes to maintain the pH of the whole pharmaceutical composition between 3.5. and 7.5, preferably between 4.0 and 7.0, and more preferably between 4.5 and 6.5, when the pharmaceutical composition containing 5 mg of active ingredient is dissolved in 100ml of water. Best results have been obtained with a buffer system having a range of pH 5.5 ± 0.5.

The pH value is measured at room temperature.

The term "buffering system" is intended to mean a compound used to resist to a change in pH upon dilution or addition of acid or alkali. Examples of buffering systems are made from pharmaceutical acceptable salts of phosphate, citrate, tartrate, acetate, fumarate, gluconate, or made from with their respective related acids and used as such or in combination with their respective related acid/salt, or mixtures thereof. Best results have been obtained with buffers of sodium citrate and with buffers of sodium citrate combined with its related acids. If salts or acids are used as such, the pH can be adjusted to the target value with appropriate, pharmaceutically accepted, acids or bases,so to create the buffer system.

Usually, the active granule according to the present invention is buffered at a concentration ranging from 1.10⁻⁴ mol/l to 1.10⁻² mol/l when the pharmaceutical composition containing 5 mg of active ingredient is dissolved in 100ml of water. This concentration corresponds to the sum of the concentrations of the different entities composing the buffering system.

Particularly, the active granule is buffered at a concentration ranging from 1.10⁻⁴ mol/l to 8.10⁻³ mol/l when the pharmaceutical composition containing 5 mg of active ingredient is dissolved in 100ml of water.

Preferably, the active granule is buffered at a concentration ranging from 2.10⁻⁴ mol/l to 5.10³ mol/l when the pharmaceutical composition containing 5 mg of active ingredient is dissolved in 100ml of water.

In a specific embodiment, the active granules contain at least an additional water soluble excipient. It is usually selected from water soluble polymer, used as binder, from cyclodextrin, used as taste masking agent and/or as binder or mixtures thereof.

The term " water soluble excipient" means an excipient at least sparingly soluble in water according to the European Pharmacopeia 7^{th} edition definition, at room temperature under atmospheric pressure.

A water soluble polymer is here understood as a polymer having a solubility superior to 5mg /ml at room temperature under atmospheric pressure.

The water soluble polymer is selected among pharmaceutically acceptable polymer binders.

The term "binder" as used herein is defined as an agent able to bind individual particles together during a granulation process, and allowing to keep the cohesion of the particles after drying. The binder may be present in the form of a single compound or in the form of a mixture of compounds.

Usually, the water soluble polymer is chosen among hydroxypropylmethyl cellulose, hydroxypropyl cellulose, polyvinylpyrrolidinone, copolymer of polyvinylpyrrolidinone and vinyl acetate; polyvinyl acetate, polyvinylalcohol or mixture thereof.

Preferably it is hydroxypropylmethyl cellulose and hydroxypropylcellulose.

Usually, the weight ratio between the water soluble polymer and the active granule is comprised between 0 and 10%. Particularly, the weight ratio between the polymer and the active granule is comprised between 0 and 5%. Preferably, the weight ratio between the polymer and the active granule is comprised between 0 and 2%.

Usually, cyclodextrin is used as taste masking agent. It is chosen among alpha cyclodextrin, β-cyclodextrin, hydroxypropyl-β-cyclodextrin, methyl-β-cyclodextrin sulfo alkyl ether cyclodextrin, gamma-cyclodextrin or mixture thereof.

Preferably, it is β-cyclodextrin, methyl-β-cyclodextrin and hydroxypropyl-β-cyclodextrin.

Usually, the molar ratio between cyclodextrin and the active ingredient is comprised between 0 and 6.

Particularly, the molar ratio between cyclodextrin and the active ingredient is comprised between 0 and 5. Preferably, the molar ratio between cyclodextrin and the active ingredient is comprised between 0 and 4.

The pharmaceutical composition of the invention contains active granules, usually an unique formulation of active granules.

Preferably, the composition of the invention does not contain any granule, produced as part of the manufacturing process of the composition which does not comprise an active ingredient. All active granules produced as part of the manufacturing process of the pharmaceutical composition do comprise a polyol fraction comprising one or more solid water soluble polyols having a molecular weight below 950 g/ mol, with the exception to lactose. The pharmaceutical composition of the invention does not contain any active granule produced as part of the manufacturing process of the pharmaceutical composition which does not comprise a buffering system. Preferably, all the active granules produced as part of the manufacturing process of the pharmaceutical composition comprise the active ingredient, a polyol fraction comprising one or more solid water soluble polyols having a molecular weight below 950 g/ mol, and a buffering system.

Usually, the active granule and / or the composition comprise pharmaceutically acceptable sweetener and flavouring agents as excipient. Preferably, these excipients are chosen among water soluble excipients.

Sweetening agents are usually chosen among aspartam, acesulfame of potassium, cyclamates, dextrose, fructose, acesulpham, sucralose, stevia derivatives, saccharin, saccharin sodium or mixture thereof.

Preferably, sweetening agents are chosen among aspartam, acesulfame of potassium, sucralose or stevia derivatives.

More preferred sweetener agent is acesulfame of potassium and sucralose.

Usually, the pharmaceutical composition according to the present invention comprises 0.0 to 3.0 % per weight of sweetener agent with respect to the total weight of the composition.

Preferably, the pharmaceutical composition comprises 0.0 to 2.0 % per weight of sweetener agent. More preferably, the pharmaceutical composition according to the invention comprises 0.0 to 1.0 % per weight of sweetener agent with respect to the total weight of the composition.

Usually, flavouring agents suitable for use in the present invention include essential oils and synthetic flavors such as citrus oils, fruit essences, peppermint oil, spearmint oil, clove oil, oil of wintergreen, anise and eucalyptus. Other artificial flavors known to those skilled in the art are also within the scope of this invention.

Usually, the pharmaceutical composition according to the present invention comprises 0.0 to 1.0 % per weight of flavouring agent. Preferably, the pharmaceutical composition according to the present invention comprises 0.0 to 0.5 % per weight of flavouring agent. More preferably 0.0 to 0.25 % per weight of flavouring agent.

In a specific embodiment, the active granule and / or the composition contain a processing aid agent.

Usually, the processing aid agent is chosen among flow enhancer, anti-sticking agent, antifoam, plasticizers, emulsifier, stabilizer or a mixture thereof. Particularly, the antisticking agent is chosen among talc, colloidal silicon dioxide, magnesium aluminiometasilicate (neusilin), magnesium trisilicate, starch, tribasic calcium phosphate, sodium stearyl fumarate, tricalcium phosphate, powdered cellulose, sodium bicarbonate, sodium ferrocyanide, potassium ferrocyanide, calcium ferrocyanide, bone phosphate, sodium silicates, calcium silicate, sodium aluminosilicate, potassium aluminium silicate, calcium aluminosilicate, bentonite, aluminium silicate, stearic acid, polydimethylsiloxane, mannitol, or a mixture thereof. The anti-sticking agent can also play a role of flow enhancer.

Particularly, the anti-foam agent is made of silicon or silicon derivative.

Particularly, the plasticizer is chosen among glycerol, fatty acids, phtalate, low molecular weight polyethylene glycol, citrate or a mixture thereof.

Particularly, the emulsifier is chosen among lecithin, leucin, sodium sulfo succinate, propyleneglycol alginate, cetyl palmitate, cetyl alcohol, cetostearyl alcohol, poloxamer, polyoxyl cetostearyl ether, polysorbates, sodium lauryl sulfate, vitamin E polyethylene glycol succinate, glycery monostearate, stearic acid, polyoxyethylene stearate, propyleneglycol monolaurate, calcium stearate, glyceryl monooleate, polyvinyl alcohol, polyoxylglycerides, sucrose palmitate, polyoxyethylene alkyl ethers or mixture thereof.

Particularly, the stabilizer is chosen among gellan gum, gelatin, propyleneglycol alginate, crospovidone, propylene glycol, aluminium stearate, sodium alginate, inulin, pectin, albumin, sucrose stearate, arginine, proline, ascorbylpalmitate,

triethanolamine, myristylalcohol, glyceryl monooleate, butylated hydroxianizole, trehalose or mixture thereof.

Optionally, the composition comprises coloring agents.

In a specific embodiment, in addition to the water soluble polyol, the active granule and / or composition contain another diluent.

A diluent is defined as a pharmaceutically acceptable excipient that provides bulk and enables accurate dosing for low dosed pharmaceutical compositions.

Particularly, the diluent is chosen among calcium phosphate, microcrystalline cellulose, sorbitol, xylitol, maltitol, dextrose, sucrose, mannitol, maltose, isomalt, lactose, maltodextrin, starch, calcium carbonate, or mixture thereof.

Usually, the active granule and / or the composition contain a diluent in an amount from 0 % to 75 % , preferably from 0 to 50 % with respect of the total weight of the granule or composition.

In a specific embodiment, the active granule and / or composition may contain a desintegrant.

A desintegrant is defined as a pharmaceutically acceptable excipient that swells in contact with water and helps to the dispersion and to the solubilization of the formulation.

Particularly, the disintegrant is chosen among sodium starch glycolate, low substituted hydroxypropylcellulose, crosslinked polyvinylpyrrolidinone, croscarmellose sodium, microcrystalline cellulose, or mixture thereof.

Usually, the active granule and / or the composition contain a disintegrant in an amount from 0 % to 15 % , preferably from 0 to 10 % with respect of the total weight of the granule or composition.

Moreover, additional intra and extra-granular pharmaceutically acceptable excipients may be added to the pharmaceutical composition.

In a specific embodiment, the composition and / or the active granule according to the present invention may contain one or more outer coatings, i.e. a final water soluble coating layer that could be applied onto the active granule or onto the whole final composition. The coating compositions are known by the man skilled in the art.

Usually, the amount of outer coating is comprised between 0 and 25% of the total weight of the pharmaceutical composition or of the active granule.

Preferably, the amount of outer coating is comprised between 0 and 20% of the total weight of the pharmaceutical composition or of the active granule.

In a particular embodiment, the outer coating contains pharmaceutically acceptable polymers such as hydroxypropylmethyl cellulose, hydroxypropyl cellulose, polyvinylpyrrolidinone, copolymer of polyvinylpyrrolidinone and vinyl acetate, polyvinyl acetate, polyvinylalcohol or mixture thereof. Preferably, it is hydroxypropylmethyl cellulose and hydroxypropyl cellulose.

In a specific embodiment of the present invention, the pharmaceutical composition contains active granules in an amount of 25 to 100% with respect of the total weight of the composition, these said active granules comprising
- 0.1 to 2.0 % of levocetirizine dihydrochloride as active ingredient, with respect of the total weight of the composition,
- at least 50 % of a solid water soluble polyol according to the claims having a molecular weight below 950 g/mol, with respect of the total weight of the composition, and
- a buffering system at a concentration ranging from 1.10⁻⁴ mol/l to 1.10⁻² mol/l of buffering system, when the pharmaceutical composition containing 5 mg of active ingredient is dissolved in 100ml of water, which contributes to maintain the pH of the pharmaceutical composition between 4.0 and 7.0.

In a specific embodiment of the present invention, the pharmaceutical composition contains active granules in an amount of 25 to 100% with respect of the total weight of the composition, these active granules comprising
- 0.1 to 1.5 % of levocetirizine dihydrochloride as active ingredient, with respect of the total weight of the composition;
- at least 50 % of a solid water soluble polyol according to the claims having a molecular weight below 950 g/mol, with respect of the total weight of the composition;
- a buffering system at a concentration ranging from 1.10⁻⁴ mol/l to 1.10⁻² mol/l of buffering system, when the pharmaceutical composition containing 5 mg of active ingredient is dissolved in 100ml of water, which contributes to maintain the pH of the pharmaceutical composition between 4.0 and 7.0; and
- a water soluble excipient selected among a cyclodextrin, the molar ratio between cyclodextrin and the active ingredient being between 0 to 6; a water soluble polymer, the weight ratio between the polymer and the granule being between 0 to 10 %; or mixture thereof.

In a specific embodiment, the pharmaceutical composition contains active granules in an amount of 50 to 100% with respect of the total weight of the composition, these active granules comprising
- 0.1 to 1.0 % of levocetirizine as active ingredient, with respect of the total weight of the composition,
- at least 70 % of a solid water soluble polyol according to the claims having a molecular weight below 350 g/mol, with respect of the total weight of the composition,
- a buffering system at a concentration ranging from 2.10⁻⁴ mol/l to 5.10³ mol/l of buffering system, when the pharmaceutical composition containing 5 mg of active ingredient is dissolved in 100ml of water,which contributes to maintain the pH of the pharmaceutical composition in a range of pH 5.5 ± 0.5,
- a water soluble excipient selected among a cyclodextrin, the molar ratio between cyclodextrin and the active ingredient being between 0 to 4; a water soluble polymer, the weight ratio between the polymer and the granule being between 0 to 2 %; or mixture thereof.

In a specific embodiment of the present invention, the pharmaceutical composition contains active granules in an amount of 25 to 100% with respect of the total weight of the composition, these active granules comprising
- 0.1 to 1.0 % of levocetirizine dihydrochloride as active ingredient, with respect of the total weight of the composition;
- at least 50 % of a solid water soluble polyol according to the claims having a molecular weight below 950 g/mol, with respect of the total weight of the composition;
- a buffering system at a concentration ranging from 1.10⁻⁴ mol/l to 1.10⁻² mol/l of buffering system, when the pharmaceutical composition containing 5 mg of active ingredient is dissolved in 100ml of water, which contributes to maintain the pH of the pharmaceutical composition between 4.0 and 7.0; and
- a water soluble excipient selected among a cyclodextrin, the molar ratio between cyclodextrin and the active ingredient being between 0 to 6; a water soluble polymer, the weight ratio between the polymer and the granule being between 0 to 10 %; or mixture thereof.

In a specific embodiment of the present invention, the pharmaceutical composition contains active granules in an amount of 50 to 100% with respect of the total weight of the composition, these active granules comprising
- 0.1 to 2.0 % of levocetirizine dihydrochloride as active ingredient, with respect of the total weight of the composition;
- at least 60 % of a solid water soluble polyol according to the claims having a molecular weight below 950 g/mol, with respect of the total weight of the composition;
- a buffering system at a concentration ranging from 1.10⁻⁴ mol/l to 8.10⁻³ mol/l of buffering system, when the pharmaceutical composition containing 5 mg of active ingredient is dissolved in 100ml of water, which contributes to maintain the pH of the pharmaceutical composition between 4.5 and 6.5;
- a water soluble excipient selected among a cyclodextrin, the molar ratio between cyclodextrin and the active ingredient being between 0 to 5; a water soluble polymer, the weight ratio between the polymer and the granule being between 0 to 5 %; or mixture thereof.

In a specific embodiment of the present invention, the pharmaceutical composition contains active granules in an amount of 50 to 100% with respect of the total weight of the composition, these active granules comprising
- 0.1 to 1.5 % of levocetirizine dihydrochloride as active ingredient, with respect of the total weight of the composition;
- at least 70 % of a solid water soluble polyol according to the claims having a molecular weight below 350 g/mol, with respect of the total weight of the composition;
- a buffering system at a concentration ranging from 2.10⁻⁴ mol/l to 5.10³ mol/l of buffering system, when the pharmaceutical composition containing 5 mg of active ingredient is dissolved in 100ml of water, which contributes to maintain the pH of the pharmaceutical composition between 4.5 and 6.5;
- a water soluble excipient selected among a cyclodextrin, the molar ratio between cyclodextrin and the active ingredient being between 0 to 4; a water soluble polymer, the weight ratio between the polymer and the granule being between 0 to 2 % ; or mixture thereof.

In a specific embodiment of the present invention, the pharmaceutical composition contains active granules in an amount of 50 to 100% with respect of the total weight of the composition, these active granules comprising
- 0.1 to 1.0 % of levocetirizine dihydrochloride as active ingredient, with respect of the total weight of the composition;
- at least 70 % of a solid water soluble polyol according to the claims having a molecular weight below 350 g/mol, with respect of the total weight of the composition;
- a buffering system at a concentration ranging from 2.10⁻⁴ mol/l to 5.10³ mol/l of buffering system, when the pharmaceutical composition containing 5 mg of active ingredient is dissolved in 100ml of water,which contributes to maintain the pH of the pharmaceutical composition in a range of pH 5.5 ± 0.5;
- a water soluble excipient selected among a cyclodextrin, the molar ratio between cyclodextrin and the active ingredient being between 0 to 4; a water soluble polymer, the weight ratio between the polymer and the granule being between 0 to 2 %; or mixture thereof.

In a specific embodiment of the present invention, the pharmaceutical composition contains active granules in an amount of 50 to 100% with respect of the total weight of the composition, these active granules comprising
- 0.1 to 1.0 % of levocetirizine dihydrochloride as active ingredient, with respect of the total weight of the composition,
- at least 70 % of mannitol, with respect of the total weight of the composition,
- a buffering system at a concentration ranging from 2.10⁻⁴ mol/l to 5.10³ mol/l of buffering system, when the pharmaceutical composition containing 5 mg of active ingredient is dissolved in 100ml of water,which contributes to maintain the pH of the pharmaceutical composition in a range of pH 5.5 ± 0.5,
- a water soluble excipient selected among a cyclodextrin, the molar ratio between cyclodextrin and the active ingredient being between 0 to 4; a water soluble polymer, the weight ratio between the polymer and the granule being between 0 to 2 % ; or mixture thereof;
- a sweetener; and
- a flavouring agent.

In a specific embodiment of the present invention, the pharmaceutical composition contains active granules in an amount of 50 to 100% with respect of the total weight of the composition, these active granules comprising
- 0.1 to 1.0 % of levocetirizine dihydrochloride as active ingredient, with respect of the total weight of the composition;
- at least 70 % of mannitol, with respect of the total weight of the composition;
- sodium citrate , citric acid or mixture thereof, as a buffering system at a concentration ranging from 2.10⁻⁴ mol/l to 5.10³ mol/l of buffering system, when the pharmaceutical composition containing 5 mg of active ingredient is dissolved in 100ml of water,which contributes to maintain the pH of the pharmaceutical composition in a range of pH 5.5 ± 0.5;
- β- cyclodextrin, as a water soluble excipient, the molar ratio between cyclodextrin and the active ingredient being between 0 to 4;
- a sweetener; and
- a flavouring agent.

In a specific embodiment of the present invention, the pharmaceutical composition contains active granules, in an amount of 25 to 100% with respect of the total weight of the composition, and an extra-granular part, these active granules comprising
- 0.1 to 1.0 % of levocetirizine dihydrochloride as active ingredient, with respect of the total weight of the composition;
- at least 50 % of a solid water soluble polyol according to the claims having a molecular weight below 950 g/mol, with respect of the total weight of the composition; and
- a buffering system at a concentration ranging from 1.10⁻⁴ mol/l to 1.10⁻² mol/l of buffering system, when the pharmaceutical composition containing 5 mg of active ingredient is dissolved in 100ml of water,which contributes to maintain the pH of the pharmaceutical composition between 4.0 and 7.0;
- a sweetener; and the extra-granular part comprising a flavouring agent.

In a specific embodiment of the present invention, the pharmaceutical composition contains active granules in an amount of 25 to 100% with respect of the total weight of the composition, and an extra-granular part, these active granules comprising
- 0.1 to 1.0 % of levocetirizine dihydrochloride as active ingredient, with respect of the total weight of the composition,
- at least 50 % of a solid water soluble polyol according to the claims having a molecular weight below 950 g/mol, with respect of the total weight of the composition, and
- a buffering system at a concentration ranging from 1.10⁻⁴ mol/l to 1.10⁻² mol/l of buffering system, when the pharmaceutical composition containing 5 mg of active ingredient is dissolved in 100ml of water,which contributes to maintain the pH of the pharmaceutical composition between 4.0 and 7.0;
   the extra-granular part comprising
- a diluent,
- a sweetener, and
- a flavouring agent.

In a specific embodiment of the present invention, the pharmaceutical composition contains active granules in an amount of 50 to 100% with respect of the total weight of the composition, and an extra-granular part, these active granules comprising
- 0.1 to 1.0 % of levocetirizine dihydrochloride as active ingredient, with respect of the total weight of the composition,
- at least 70 % of a solid water soluble polyol according to the claims having a molecular weight below 950 g/mol, with respect of the total weight of the composition, and
- a buffering system at a concentration ranging from 2.10⁻⁴ mol/l to 5.10³ mol/l of buffering system, when the pharmaceutical composition containing 5 mg of active ingredient is dissolved in 100ml of water,which contributes to maintain the pH of the pharmaceutical composition in a range of pH 5.5 ± 0.5
- β- cyclodextrin, as a water soluble excipient, the molar ratio between cyclodextrin and the active ingredient being between 0 to 4,
- a water soluble polymer, the weight ratio between the polymer and the granule being between 0 to 2 %,
- a sweetener;
   the extra-granular part comprising
- a flow enhancing agent,
- a flavouring agent, and
- a diluent.

In one embodiment, the active granule is composed by a core coated by at least two coatings, the first one being used to isolate the core from the active ingredient, being the second coating .

In a specific embodiment, the polyol fraction is included in a coated core.

In this specific embodiment, the present invention provides a pharmaceutical composition, in a solid form, allowing an oral administration of an unit dose ranging from 0.50 mg to 25.00 mg of levocetirizine dihydrochloride, as active ingredient, and containing active granules, said active granules comprising
- a core which comprises at least a solid water soluble polyol fraction having a molecular weight below 950 g/mol; the polyol fraction comprising mannitol;
- a first coating applied onto the core, and comprising at least an excipient; and
- a second coating applied onto the first coating, and comprising the active ingredient and a buffering system.

Preferably, the active granules are produced as part of the manufacturing process of the pharmaceutical composition.

The core contains at least 50% of polyol fraction; usually at least 60 % of polyol fraction and preferably at least 70% of polyol fraction with respect of the total weight of the core.

In a specific embodiment, the core comprises other pharmaceutically acceptable excipients, such as diluent, sweetener, aroma or other pharmaceutically acceptable excipients known by the man skilled in the art, or mixture thereof. Preferably, these excipients are chosen among water soluble excipients.

Usually, the core yields for 50 to 99 % per weight with respect to the total weight of the granule. Particularly, the core yields for 60 to 98 % per weight with respect to the total weight of the granule. Preferably, the core yields for 70 to 97 % per weight with respect to the total weight of the granule.

Preferably, the core does not comprise any active ingredient. Preferably, the core does not comprise any other drug.

The first coating is designed to isolate the core from the second coating. The first coating comprises at least an excipient.

The excipient of the first coating is selected from water soluble polymer, polymer dispersion, cyclodextrin, and mixtures thereof. Preferably, the excipient is cyclodextrin or water soluble polymer.

A water soluble polymer is here understood as a polymer having a solubility superior to 5mg /ml at room temperature under atmospheric pressure.

Usually, the water soluble polymer is chosen among hydroxypropylmethyl cellulose, hydroxypropyl cellulose, polyvinylpyrrolidinone, copolymer of polyvinylpyrrolidinone and vinyl acetate, polyvinyl acetate, polyvinylalcohol or mixture thereof. Preferably, it is hydroxypropylmethyl cellulose and hydroxypropyl cellulose.

The polymer dispersion is here understood as a material comprising more than one phase where at least one of the phase consists of finely divided polymers dispersed throughout a continuous phase as a liquid.

Usually, the polymer dispersion is chosen among polyacrylate, polymethacrylate copolymers, and mixture thereof.

Usually, cyclodextrin is used as taste masking agent. It is chosen among alpha cyclodextrin, beta-cyclodextrin, hydroxypropyl-beta-cyclodextrin, methyl-beta-cyclodextrin sulfo alkyl ether cyclodextrin, gamma-cyclodextrin or mixture thereof.

Preferably, it is beta-cyclodextrin, methyl-beta-cyclodextrin and hydroxypropyl-beta-cyclodextrin.

Usually, the molar ratio between cyclodextrin from the first coating and the active ingredient is comprised between 0 and 6. Particularly, the molar ratio between cyclodextrin from the first coating and the active ingredient is comprised between 0 and 5. Preferably, the molar ratio between cyclodextrin from the first coating and the active ingredient is comprised between 0 and 4.

Usually, the weight ratio between the polymer from the first coating and the core is comprised between 0 and 25. Particularly, the weight ratio of the polymer from the first coating and the core is comprised between 0 and 23. Preferably, the weight ratio of the polymer from the first coating and the core is comprised between 0 and 20.

In a specific embodiment, the first coating comprises a buffering system which contributes to maintain the pH of the first coating and of the whole pharmaceutical composition between 3.5 and 7.5, usually between 4.0 and 7.0, preferably between 4.5 and 6.5. Best results have been obtained with a buffer system having a range of pH 5.5 ± 0.5.

Usually, the first coating is buffered at a concentration ranging from 1.10⁻⁴ mol/l to 1.10⁻² mol/l when the pharmaceutical composition containing 5 mg of active ingredient is dissolved in 100ml of water. This concentration corresponds to the sum of the concentrations of the different entities composing the buffering system.

Particularly, the first coating is buffered at a concentration ranging from 1.10⁻⁴ mol/l to 8.10⁻³ mol/l when the pharmaceutical composition containing 5 mg of active ingredient is dissolved in 100ml of water.

Preferably, the first coating is buffered at a concentration ranging from 2.10⁻⁴ mol/l to 5.10³ mol/l when the pharmaceutical composition containing 5 mg of active ingredient is dissolved in 100ml of water. In a specific embodiment, the first coating and/or the second coating and/or the composition contain a processing aid agent.

Usually, the processing aid agent is chosen among flow enhancers, anti-sticking agent, antifoam, plasticizers, emulsifier, stabilizer and mixture thereof.

Usually, the first coating represents 0% to 40 % per weight with respect to the total weight of the granule. Particularly, the first coating represents 0% to 30 % per weight with respect to the total weight of the granule. The first coating represents preferably 0% to 25 % per weight with respect to the total weight of the granule.

Optionally, the first coating comprises additional excipients, such as sweeteners, aroma, coloring agent.

Preferably, the first coating does not comprise any active ingredient. Preferably, the first coating does not comprise any other drug.

The second coating comprises the active ingredient.

Usually, the second coating according to the present invention represents 0.5% to 40 % per weight with respect to the total weight the granule. Particularly, the second coating according to the present invention represents 1 % to 30 % per weight with respect to the total weight of the granule. The second coating according to the present invention represents preferably 1.0 % to 20 % per weight with respect to the total weight of the granule.

The second coating comprises a buffering system which contributes to maintain the pH of the second coating and of the whole pharmaceutical composition between 3.5 and 7.5, usually between 4.0 and 7.0, preferably between 4.5 and 6.5. Best results have been obtained with a buffer having a range of pH 5.5 ± 0.5.

Usually, the second coating according to the present invention is buffered at a concentration ranging from 1.10⁻⁴ mol/l to 1.10⁻² mol/l when the composition containing 5 mg of active ingredient is dissolved in 100ml of water. Particularly, the second coating is buffered at a concentration ranging from 1.10⁻⁴ mol/l to 8.10⁻³ mol/l when the composition containing 5 mg of active ingredient is dissolved in 100ml of water. Preferably, the second coating is buffered at a concentration ranging from 2.10⁻⁴ mol/l to 5.10³ mol/l when the composition containing 5 mg of active ingredient is dissolved in 100ml of water.

Usually, the second coating comprises 0.1 to 4 % per weight of active ingredient with respect to the total weight of the pharmaceutical composition.

Usually, the second coating comprises at least a water soluble pharmaceutically acceptable excipient.

Usually , the pharmaceutically acceptable excipient is a cyclodextrin, a water soluble polymer, used as binder, and optionally another pharmaceutically acceptable excipient known by the man skilled in the art or a mixture thereof.

Usually, cyclodextrin is used as taste masking agent. It is chosen among alpha cyclodextrin, beta-cyclodextrin, hydroxypropyl-beta-cyclodextrin, methyl-beta-cyclodextrin sulfo alkyl ether cyclodextrin, gamma-cyclodextrin or mixture thereof.

Preferably, it is beta-cyclodextrin, methyl-beta-cyclodextrin and hydroxypropyl-beta-cyclodextrin.

Usually, the molar ratio between cyclodextrin from the second coating and the active ingredient is comprised between 0 and 6. Particularly, the molar ratio between cyclodextrin from the second coating and the active ingredient is comprised between 0 and 5. Preferably, the molar ratio between cyclodextrin from the second coating and the active ingredient is comprised between 0 and 4.

Usually, the water soluble polymer is chosen among hydroxypropylmethyl cellulose, hydroxypropyl cellulose, polyvinylpyrrolidinone, copolymer of polyvinylpyrrolidinone and vinyl acetate; polyvinyl acetate, polyvinylalcohol or mixture thereof.

Preferably it is hydroxypropylmethyl cellulose and hydroxypropylcellulose.

Usually, the weight ratio between the water soluble polymer and the granule is comprised between 0 and 10%. Particularly, the weight ratio between the polymer and the granule is comprised between 0 and 5%. Preferably, the weight ratio between the polymer and the granule is comprised between 0 and 2%.

Optionally, the second coating comprises process aid-agent, sweetening agent, flavor, colorant or mixtures thereof. Example of process aid-agents are described above.

Optionally, the second coating comprises sweetening agents.

Sweetening agents are usually chosen among aspartam, acesulfame of potassium, cyclamates, sucralose, stevia derivatives, saccharin, saccharin sodium or mixture thereof.

Preferably, sweetening agents are chosen among aspartam, acesulfame of potassium, sucralose or stevia derivatives.

More preferred sweetener agent is acesulfame of potassium and sucralose.

Usually, the pharmaceutical composition according to the present invention comprises 0.0 to 3.0 % per weight of sweetener agent with respect to the total weight of the composition.

Preferably, the pharmaceutical composition comprises 0.0 to 2.0 % per weight of sweetener agent. More preferably, the pharmaceutical composition according to the invention comprises 0.0 to 1.0 % per weight of sweetener agent with respect to the total weight of the composition.

Usually, flavouring agents suitable for use in the present invention include essential oils and synthetic flavors such as citrus oils, fruit essences, peppermint oil, spearmint oil, clove oil, oil of wintergreen, anise, eucalyptus and the like. Other artificial flavors known to those skilled in the art are also within the scope of this invention.

Usually, the pharmaceutical composition according to the present invention comprises 0.0 to 1.0 % per weight of flavouring agent. Preferably, the pharmaceutical composition according to the present invention comprises 0.0 to 0.5 % per weight of flavouring agent. More preferably 0.0 to 0.25 % per weight of flavouring agent.

In a specific embodiment of the invention, the pharmaceutical composition allowing an oral administration of an unit dose ranging from 1.00 mg to 10.00 mg of levocetirizine dihydrochloride, as active ingredient, comprises
- a core which comprises at least mannitol;
- a first coating applied onto the core, and comprising at least beta-cyclodextrin, the molar ratio between cyclodextrin and levocetirizine being

comprised between 0 and 3, and comprising also a buffering system which contributes to maintain the pH between 4.5 and 6.5, when the pharmaceutical composition containing 5 mg of active ingredient is dissolved in 100ml of water;
- a second coating applied onto the first coating, and comprising levocetirizine at least a water soluble excipient, and a buffering system which contributes to maintain the pH between 4.5 and 6.5, when the pharmaceutical composition containing 5 mg of active ingredient is dissolved in 100ml of water; and
- optionally, a final water soluble coating applied onto the second coating.

In a specific embodiment of the invention, the pharmaceutical composition comprises
- a core which comprises at least mannitol;
- a first coating applied onto the core, and comprising at least hydroxypropyl methylcellulose or hydroxypropyl cellulose; and
- a second coating applied onto the first coating, and comprising levocetirizine and at least cyclodextrin. and comprising also a buffering system which contributes to maintain the pH between 4.5 and 6.5, when the pharmaceutical composition containing 5 mg of active ingredient is dissolved in 100ml of water.

In a specific embodiment, the composition and / or the granule according to the present invention may contain one or more outer coatings, i.e. a final water soluble coating layer. The coating compositions are known by the man skilled in the art.

Usually, the amount of outer coating is comprised between 0 and 25% of the total weight of the pharmaceutical composition or of the granule. Preferably, the amount of outer coating is comprised between 0 and 20% of the total weight of the pharmaceutical composition or of the granule.

In one specific embodiment of the invention, the pharmaceutical composition allows an oral administration of an unit dose ranging from 0.5 mg to 25 mg of levocetirizine dihydrochloride, as active ingredient and contains active granules in an amount of 25% to100% with respect to the total weight of the composition, these active granules comprising
- a core which comprises at least a solid water soluble polyol according to the claims having a molecular weight below 950 g/mol, said core yields for 60 to 99 % per weight with respect to the total weight the granule,
- a first coating applied onto the core, and comprising at least a principal excipient, said first coating represents 1% to 40 % per weight with respect to the total weight of the active granule,
- a second coating applied onto the first coating, and comprising levocetirizine dihydrochloride and a buffering system; said second coating represents 0.5% to 40 % per weight with respect to the total weight of the active granule, and levocetirizine dihydrochloride represents 0.1 to 2 % with respect of the total weight of the composition; and
- optionally, a final water soluble coating applied onto the second coating. Particularly, the pharmaceutical composition allows an oral administration of an unit dose ranging from 1 mg to 10 mg of levocetirizine dihydrochloride, as active ingredient and contains active granules in an amount of 25% to 100% with respect to the total weight of the composition, these active granules comprising
- a core,which comprises at least mannitol, which core yields for 50 to 99 % per weight with respect to the total weight of the active granule,
- a first coating applied onto the core, and comprising as principal excipient a cyclodextrin, the molar ratio between cyclodextrin and levocetirizine dihydrochloride being comprised between 0 and 6, or a water soluble polymer; the weight ratio between the polymer from the first coating and the core being comprised between 0 and 15, and optionally comprising a buffering system which contributes to maintain the pH between 4.0 and 7.0; said first coating represents 1% to 40 % per weight with respect to the total weight of the active granule,
- a second coating applied onto the first coating, and comprising levocetirizine dihydrochloride and a buffering system which contributes to maintain the pH between 4.0 and 7.0; said second coating represents 0.5% to 40 % per weight with respect to the total weight of the granule, and levocetirizine dihydrochloride represents 0.1 to 1 % with respect of the total weight of the composition and
- optionally, a final water soluble coating applied onto the second coating.

In a preferred embodiment of the invention, the pharmaceutical composition allows an oral administration of an unit dose ranging from 1 mg to 10 mg of levocetirizine dihydrochloride, as active ingredient and contains active granules in an amount of 50% to100% with respect to the total weight of the composition, these active granules comprising
- a core which comprises at least mannitol,and representing 70 to 97 % per weight with respect to the total weight the granule,
- a first coating applied onto the core, and comprising as principal excipient a cyclodextrin, the molar ratio between cyclodextrin and levocetirizine dihydrochloride being comprised between 0 and 4, or a water soluble polymer; the weight ratio between the polymer from the first coating and the core being comprised between 0 and 10, and optionally comprising also a buffering system which contributes to maintain the pH between 5.0 and 6.0; said first coating represents 1 % to 25 % per weight with respect to the total weight of the active granule,
- a second coating applied onto the first coating, and comprising levocetirizine dihydrochloride and a buffering system which contributes to maintain the pH between 5.0 and 6.0; said second coating represents 1% to 20 % per weight with respect to the total weight the active granule, and levocetirizine dihydrochloride represents 0.1 to 1 % with respect of the total weight of the composition; and
- optionally,a sweetener.
Optionally, a final water soluble coating applied onto the second coating.
Optionally, the extra-granular part comprises a flow enhancing agent, a diluent and/or a flavouring agent.

The present invention concerns also a process for preparing the pharmaceutical composition. Acid, such as HCl, or base, such as NaOH, can be used to obtain the suitable buffer system.

The composition can be further processed to obtain various oral forms, including tablets, orally disintegrating tablets and capsules.

The composition is not processed to obtain effervescent tablets. Preferably, it does not comprise any agents that generate effervescence, such as combination of anhydrous citric acid/ tartric acids and sodium bicarbonate, which in contact with water makes carbon dioxide and produces effervescence. The pharmaceutical composition of the invention is a non-effervescent form.

The tablet may be chewable or destined to be crunched or sucked.

The compositions can also be used as such and could be named either as dry syrup or as granulate.

Preferably, the pharmaceutical composition of the invention is an orally disintegrating tablet or a granulate being placed in an appropriate packaging system such as capsule, sachet or bottle.

A dry syrup or granulate is defined as a solid composition, such as for example granules, designed to be administered orally in this form or after addition to a liquid. This kind of formulation offers some advantages to patient having difficulties to swallow monolithic dosage forms, such as tablets, and offers high dosing and administration flexibility.

Another advantage of the invention is that the pharmaceutical composition in a granular form, once placed onto the tongue disperses almost immediately, without having a gritty or other noticeable residue.

An orodispersible tablet is defined as a tablet to be placed in the mouth where it disperses rapidly before swallowing. Orodispersible tablets are solid unit dosage forms, which disintegrate in the mouth within a very short period of time in the presence of saliva.
Orodispersible tablets disintegrate within 3 min when examined by the pharmacopeia test for disintegration of tablets and capsules.

Tablets and capsules could be made from the granules and optionally with some additional pharmaceutical acceptable excipients, like processing aid agents, diluent, sweetener, flavoring agent. The tablets could optionally be coated.

The granules are obtained by usual technologies, such as fluid bed granulation, fluid bed coating, high shear granulation, high shear coating, spray drying, melt granulation, wet granulation by extrusion. Tablets made from granules could be obtained by direct tableting. The technologies are known by the man skilled in the art.

In a particular embodiment, the present invention relates to a granule obtained by a wet coating process of the first coating layer onto the core. The material from the first coating layer being dissolved or suspended in an appropriate coating solvent, sprayed onto the solid core material and subsequently dried to form an intermediate granule, and of the subsequent coating of the second coating layer onto the intermediate granule . The material from the second coating,a buffered solution containing the active ingredient, being dissolved or suspended in an appropriate coating solvent prior to coating.

In a particular embodiment, the present invention relates to an active granule obtained by fluid bed granulation or fluid bed coating. The active ingredient being dissolved in an appropriate buffered solvent, sprayed onto the solid polyol and subsequently dried to form a granule.

Usually, appropriate solvents are water, ethanol, isopropanol, dichloromethane, acetone or a mixture thereof. Preferably, the solvent is water.

In another particular embodiment, the present invention relates to a granule obtained by spray drying process. The active ingredient and the polyol being dissolved in an appropriate buffered solvent, and spray dried to form a granule.

Usually, appropriate solvents are water, ethanol, isopropanol, dicholromethane, acetone or a mix thereof. Preferably, the solvent is water.

In a specific embodiment, the granule is subsequently densified by dry compaction or any technique known by the man skilled in the art.

In a particular embodiment, the present invention provides an unit dose pharmaceutical composition completely soluble within less than 2 minutes in a recipient containing about 50 ml of water at room temperature or leads to a translucid homogeneous suspension.

The pharmaceutical composition of the invention is in a form of homogeneous granules, or in a tablet containing those granules or in a capsule containing those granules.

One advantage of the invention is that active ingredient is homogeneously dispersed within the granule, as there is one type of granules in the pharmaceutical composition.

Another advantage of the invention is that the pharmaceutical composition has an acceptable taste.

Another advantage of the invention is that the pharmaceutical composition has an appropriate stability, i.e. the active ingredient is not degraded. One advantage of the invention is that the unit dose, an amount of the pharmaceutical composition suitable for the administration of the required dose of active ingredient, corresponds to a minimum amount of 50 mg of pharmaceutical composition, and preferably 100 mg.

One advantage of the invention is that the unit dose pharmaceutical composition is completely solubilized or dispersed within less than 2 minutes in a glass containing about 50 ml of water at room temperature.

Another advantage of the invention is that the pharmaceutical composition has in the same time quick dissolution of the active ingredient into water.

The composition of the invention could be taken directly into the mouth, dispersed into food or aqueous liquid and swallowed without bitter taste.

Another advantage of the invention is that the pharmaceutical composition, once placed onto the tongue disperses almost immediately, without having a gritty or other noticeable residue.

In the following examples, the term « intra-granular phase » means the fraction of the pharmaceutical composition containing the active granules. The term « extra-granular phase » means the fraction of the pharmaceutical composition which does not contain the active granules.

### Example 1

An aqueous solution of levocetirizine dihydrochloride, β-cyclodextrine, acesulfame K, Na citrate, mannitol and flavor were prepared according to table 1. The pH of the solution was adjusted to 5.5 with HCl. Then the composition were prepared by spray drying of the aqueous solution.
The obtained granules were further densified by dry compaction.
The content of active material and the stability were assessed in different conditions. The results are given in Table 2.
It can be seen that the granules have a good homogeneity and no degradation of levocetirizine could be observed.
Dissolution of a therapeutic dose of 5 mg of levocetirizine dihydrochloride in 50 ml of water took less than 2 minutes.

**Table 1 Composition example 1**

| MATERIAL | QUANTITY % |
|---|---|
| Levocetirizine HCl | 0.326 |
| β-cyclodextrin | 2.561 |
| Acesulfame K | 0.094 |
| Trisodium citrate.2H2O | 0.796 |
| Mannitol DC 400 | 96.141 |
| Strawberry flavor | 0.082 |
| TOTAL | 100.0 |

Mannitol (Mannitol DC400) is a granulated powder mannitol having an average particle size of about 300 - 400 µm, Na citrate (trisodiumcitrate) and HCl are used as buffering agent, Acesulfame K (acesulfame potassium) is used as sweetner, β-cyclodextrine is used as taste masking agent, Strawberry flavor is used as flavoring agent.

**Table 2 Stability results example 1**

| Time | Content in active drug (%) | |
|---|---|---|
| | 40°C/75%HR | 25°C/60%HR |
| 0 | 101,45 ± 2,95 | 98,14 ± 2,86 |
| 2 weeks | 101,05 ± 0,33 | 108,30 ± 1,47 |
| 8 weeks | 104,59 ± 2,05 | 104,2 ± 1,77 |

As shown in experimental results of Table 2, the obtained composition was stable and homogenous.
The obtained composition had no bitter taste.

### Example 2

The composition containing levocetirizine dihydrochloride were prepared by fluid bed granulation process.
All materials except mannitol and strawberry flavors were dissolved in water and granulated with mannitol and then dried. The pH of the solution was about 6.0 After drying, the granules were mixed with strawberry flavor.

**Table 3 Composition example 2**

| MATERIAL | QUANTITY % |
|---|---|
| Levocetirizine HCl | 0.31 |
| Trisodium citrate.2H2O | 3.2 |
| Mannitol DC 300 | q.s. |
| Acesulfame K | 0.37 |
| Strawberry flavor | 0.1 |
| B-cyclodextrin | 2.5 |
| Total | 100 |

Mannitol (Mannitol DC300) is a granulated powder mannitol having an average particle size of about 250 - 350 µm. Trisodium Critrate is used as buffering agent to obtain a pH 6.0.

The abbreviation q.s. means "quantum satis" , adding enough mannitol to achieve the total weight.

Acesulfame K (acesulfame potassium) is used as sweetner. β-cyclodextrine is used as taste masking agent. Strawberry flavor is used as flavoring agent.

The content of active material and the stability were assessed in different conditions. The obtained composition was stable and homogeneous. It had an acceptable taste and palatability. The obtained composition had no bitter taste.

The obtained composition was an immediate release composition.

### Example 3

The composition containing levocetirizine dihydrochloride were prepared by fluid bed granulation process.

All materials except mannitol and strawberry flavors were dissolved in water and granulated with mannitol, and then dried. The pH of the solution was 5.5. After drying, the granules were mixed with strawberry flavor.

**Table 4 Composition example 3**

| MATERIAL | QUANTITY % |
|---|---|
| Levocetirizine HCl | 0.31 |
| Trisodium citrate.2H2O | 1.5 |
| Citric acid (monohvdrate) | 0.3 |
| Mannitol DC 400 | q.s. |
| Sucralose | 0.1 |
| Strawberry flavor | 0.1 |
| beta-cyclodextrin | 1.25 |
| Total | 100 |

Mannitol (Mannitol DC400) is a granulated powder mannitol having an average particle size of about 300 - 400 µm, trisodium citrate and citric acid are used as buffering agent. sucralose is used as sweetner; β-cyclodextrine is used as taste masking agent. Strawberry flavor is used as flavoring agent. The content of active material and the stability were assessed in different conditions. The obtained composition was stable and homogeneous. It had an acceptable taste and palatability. The obtained composition had no bitter taste.

The obtained composition was an immediate release composition.

### Example 4

The composition containing levocetirizine dihydrochloride were prepared by fluid bed granulation process.
All materials except mannitol and strawberry flavors were dissolved in water and granulated with mannitol, and then dried. After drying, the granules were mixed with strawberry flavor.

**Table 5 Composition example 4**

| MATERIAL | QUANTITY % |
|---|---|
| Levocetirizine HCl | 0.31 |
| Trisodium citrate.2H2O | 3.0 |
| Citric acid (monohydrate) | 0.6 |
| hydroxypropylmethylcellulose | 3 |
| Mannitol DC 400 | q.s. |
| Sucralose | 0.1 |
| Strawberry flavor | 0.1 |
| Beta-cyclodextrin | 1.25 |
| Total | 100 |

Mannitol (Mannitol DC400) is a granulated powder mannitol having an average particle size of about 300 - 400 µm.

Trisodium citrate and citric acid are used as buffering agent. Hydroxypropylmethylcellulose (Pharmacoat® 603) is a soluble binder. Sucralose is used as sweetner. B-cyclodextrine is used as taste masking agent. Strawberry flavor is used as flavoring agent.

The content of active material and the stability were assessed in different conditions. The obtained composition was stable and homogeneous. It had an acceptable taste and palatability. The obtained composition was an immediate release composition.
1 g of dry syrup prepared in Example 4 was directly placed in the mouth of the subject and taste was evaluated on a 3-point scale. No bitterness was detected.

### Example 5

The composition containing levocetirizine dihydrochloride were prepared by fluid bed granulation process.
All materials except mannitol, anhydrous silicon dioxide and strawberry flavors were dissolved in water solution at pH 5.5 , granulated with mannitol and dried. After drying, the granules were mixed with strawberry flavor and anhydrous silicon dioxide.

**Table 6 Composition example 5**

| MATERIAL | QUANTITY % |
|---|---|
| Levocetirizine HCl | 0.30 |
| Trisodium citrate.2H2O | 2.33 |
| Citric acid (monohydrate) | 0.35 |
| hydroxypropylcellulose | 0.03 |
| Mannitol DC 300 | q.s. |
| Sucralose | 0.19 |
| Strawberry flavor | 0.1 |
| Beta-cyclodextrin | 1.13 |
| Anhydrous silicon dioxide | 0.5 |
| Total | 100 |

Mannitol (Pearlitol ®DC300) is a granulated powder mannitol having an average particle size of about 250 - 350 µm.

Trisodium citrate (hydrated) and citric acid (hydrated) are used as buffering agent. Hydroxypropyl-cellulose (Klucel ®EF) is a soluble binder. Sucralose is used as sweetner. B-cyclodextrine (Kleptose ®4PC) is used as taste masking agent. Strawberry flavor is used as flavoring agent, Anhydrous silicon dioxide (Aerosil ®200) is used as anti-caking agent

The content of active material and the stability were assessed in different conditions. The obtained composition was stable and homogeneous. It had an acceptable taste and palatability. The obtained composition was an immediate release composition.

### Example 6

The composition containing levocetirizine dihydrochloride were prepared by spray drying of an aqueous solution containing all materials. The pH of the solution was adjusted to 5.5 with HCI prior to spray drying.

The composition is according to Table 7.

The content of active material and the stability were assessed at 40°C/75% RH in a closed HDPE bottle.

The results are given in Table 8.

It can be seen that the granules had a good homogeneity and no degradation of the Levocetirizine could be observed.

Moreover, the composition was substantialy free from the taste of levocetirizine when placed on the human tongue in dry form or when dispersed in water.

**Table 7 Composition example 6**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| Beta-cyclodextrin | 2.57 |
| Total | 100 |

Mannitol (Mannitol DC400) is a granulated powder mannitol having an average particle size of about 300 - 400 µm. Trisodium citrate and HCl are used as buffering agent. Acesulfame K (acesulfame potassium) is used as sweetner. β-cyclodextrin is used as taste masking agent. Strawberry flavor is used as flavoring agent..

**Table 8 Stability results example 6**

| Content in active drug (%) - 40°C/75%RH (n=5) | | |
|---|---|---|
| T0 | T 1 Month | T 3 Months |
| 97.9 ± 0.8% | 99.0 ± 2,7% | 98.9 ± 0.7% |

As shown in experimental results of Table 8, the obtained composition was stable and homogeneous. It had an acceptable taste and palatability.

### Example 7

The composition containing levocetirizine dihydrochloride were prepared by spray drying of an aqueous solution containing all materials. The pH of the solution was adjusted to 5.5 with HCI prior to spray drying.

The compositions is according to Table 9.

The content of active material and the stability were assessed at 40°C/75% RH in a closed HDPE bottle.

The results are given in Table 10.

It can be seen that the granules had a good homogeneity and no degradation of levocetirizine could be observed.

Moreover, the composition was substantialy free from the taste of Levocetirizine when placed on the human tongue in dry form or when dispersed in water.

Dissolution of a therapeutic dose of 5 mg of levocetirizine dihydrochloride in 50 ml of water took less than 2 minutes.

**Table 9 Composition example 7**

| MATERIAL | QUANTITY % |
|---|---|
| Levocetirizine HCl | 0.16 |
| Trisodium citrate.2H2O | 0.8 |
| Mannitol DC 400 | q.s. |
| Acesulfame K | 0.1 |
| Strawberry flavor | 0.1 |
| Beta-cyclodextrin | 1.28 |
| Total | 100 |

Mannitol (Mannitol DC400) is a granulated powder mannitol having an average particle size of about 300 - 400 µm. Trisodium citrate and HCI are used as buffering agent. Acesulfame K (acesulfame potassium) is used as sweetner. β-cyclodextrin is used as taste masking agent. Strawberry flavor is used as flavoring agent.

**Table 10 Stability results example 7**

| Content in active drug (%) - 40°C/75%RH (n=5) | | |
|---|---|---|
| T0 | T 1 Month | T 3 Months |
| 100.0 ± 0.3% | 97.3 ± 0.4% | 97.7 ± 0.3% |

As shown in experimental results of Table 10, the obtained composition was stable and homogeneous. It had an acceptable taste and palatability. The obtained composition had no bitter taste.

### Example 8

Composition containing levocetirizine dihydrochloride were prepared by spray drying of an aqueous solution containing all materials. The pH of the solution was adjusted to 5.5 with HCI prior to spray drying.

The compositions is according to Table 11.

The content of active material and the stability were assessed at 40°C/75% RH in a closed HDPE bottle.

The results are given in Table 12.

It can be seen that the granules had a good homogeneity and no degradation of levocetirizine could be observed.

Moreover, the composition was substantialy free from the taste of levocetirizine when placed on the human tongue in dry form or when dispersed in water. The composition complied with the requirements.

**Table 11 composition example 8**

| MATERIAL | QUANTITY % |
|---|---|
| Levocetirizine HCl | 0.16 |
| Trisodium citrate.2H2O | 0.8 |
| Mannitol DC 400 | q.s. |
| Acesulfame K | 0.1 |
| Strawberry flavor | 0.1 |
| Beta-cyclodextrin | 1.28 |
| Total | 100 |

**Table 12 Stability results example 8**

| Content in active drug (%) - 40°C/75%RH (n=5) | | |
|---|---|---|
| T0 | T 1 Month | T 3 Months |
| 97.9 ± 0.3% | 96.8 ± 0.3% | 98.4 ± 1.0% |

As shown in experimental results of Table 12, the obtained composition was stable and homogeneous. It had an acceptable taste and palatability. The obtained composition had no bitter taste.

### Example 9

Composition containing levocetirizine dihydrochloride were prepared by spray drying of an aqueous solution containing all materials. The pH of the solution was adjusted to 5.5 with HCI prior to spray drying.

The compositions is according to Table 13.

The content of active material and the stability were assessed at 40°C/75% RH in a closed HDPE bottle.

The results are given in Table 14.

It can be seen that the granules had a good homogeneity and no degradation of levocetirizine could be observed.

Moreover, the composition was substantialy free from the taste of levocetirizine when placed on the human tongue in dry form or when dispersed in water. The composition complied with the requirements.

**Table 13 Composition example 9**

| MATERIAL | QUANTITY % |
|---|---|
| Levocetirizine HCl | 0.32 |
| Trisodium citrate.2H2O | 0.8 |
| Mannitol DC 400 | q.s. |
| Acesulfame K | 0.1 |
| Strawberry flavor | 0.1 |
| Hydroxypropyl β-cyclodextrin | 3.15 |
| Total | 100 |

**Table 14 Composition example 9**

| Content in active drug (%) - 40°C/75%RH (n=5) | | |
|---|---|---|
| T0 | T 1 Month | T 3 Months |
| 100.0 ± 0.6% | 95.8 ± 0.4% | 99.9 ± 0.6% |

As shown in experimental results of Table 14, the obtained composition was stable and homogeneous. It had an acceptable taste and palatability.

### Example 10

Composition containing levocetirizine dihydrochloride were prepared by spray drying of an aqueous solution containing all materials. The pH of the solution was adjusted to 5.5 with HCI prior to spray drying.

The compositions is according to Table 15.

The content of active material and the stability were assessed at 40°C/75% RH in a closed HDPE bottle.

The results are given in Table 16.

It can be seen that the granules had a good homogeneity and no degradation of levocetirizine could be observed.
Moreover, the composition was substantialy free from the taste of levocetirizine when placed on the human tongue in dry form or when dispersed in water. Dissolution of a therapeutic dose of 5 mg of levocetirizine dihydrochloride in 50 ml of water took less than 2 minutes.

**Table 15 Composition example 10**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| Methyl β-cyclodextrin | 3.3 |
| Total | 100 |

**Table 16 Stability results example 10**

| Content in active drug (%) - 40°C/75%RH (n=5) | | |
|---|---|---|
| T0 | T 1 Month | T 3 Months |
| 102.3 ± 0.3% | 97.8 ± 0.5% | 98.8 ± 0.6% |

As shown in experimental results of Table 16, the obtained composition was stable and homogeneous. It had an acceptable taste and palatability.

### Example 11

Composition containing levocetirizine dihydrochloride were prepared by spray drying of an aqueous solution containing all materials. The pH of the solution was adjusted to 5.5 with HCI prior to spray drying.

The compositions is according to Table 17.

The content of active material and the stability were assessed at 40°C/75% RH in a closed HDPE bottle.

The results are given in Table 18.

It can be seen that the granules had a good homogeneity and no degradation of levocetirizine could be observed.

Dissolution of a therapeutic dose of 5 mg of levocetirizine dihydrochloride in 50 ml of water took less than 2 minutes.

**Table 17 Composition example 11**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| α-cyclodextrin | 2.1 |
| Total | 100 |

**Table 18 Stability results example 11**

| Content in active drug (%) - 40°C/75%RH (n=5) | | |
|---|---|---|
| T0 | T 1 Month | T 3 Months |
| 94.5 ± 0.6% | 95.5 ± 0.7% | 99.0 ± 0.6% |

As shown in experimental results of Table 18, the obtained composition was stable and homogeneous. It had an acceptable taste and palatability.

### Example 12 (outside the scope of the claims)

Composition containing levocetirizine dihydrochloride were prepared by spray drying of an aqueous solution containing all materials. The pH of the solution was adjusted to 5.5 with HCI prior to spray drying.

The compositions is according to Table 19.

The content of active material and the stability were assessed at 40°C/75% RH in a closed HDPE bottle.

The results are given in Table 20.

It can be seen that the granules had a good homogeneity and no degradation of the Levocetirizine could be observed.

Moreover, the composition was substantialy free from the taste of Levocetirizine when placed on the human tongue in dry form or when dispersed in water.

**Table 19 Composition example 12**

| MATERIAL | QUANTITY % |
|---|---|
| Levocetirizine HCl | 0.33 |
| Trisodium citrate.2H2O | 0.8 |
| Lactose | q.s. |
| Acesulfame K | 0.1 |
| Strawberry flavor | 0.1 |
| β-cyclodextrin | 2.56 |
| Total | 100 |

Lactose is used as diluent. Sodium citrate and HCI are used as buffering agent. Acesulfame K (acesulfame potassium) is used as sweetner. β-cyclodextrin is used as taste masking agent. Strawberry flavor is used as flavoring agent.

**Table 20 Stability results example 12**

| Gontent in active drug (%) - 40°C/75%RH (n=5) | | |
|---|---|---|
| T0 | T 1 Month | T 3 Months |
| 100.1 ± 0.9% | 98.3 ± 3.0% | 108.4 ± 0.5% |

As shown in experimental results of Table 20, the obtained composition was stable and homogeneous. It had an acceptable taste and palatability.

### Example 13

Composition containing levocetirizine dihydrochloride were prepared by spray drying of an aqueous solution containing all materials. The pH of the solution was adjusted to 5.5 with HCI prior to spray drying.
The compositions is according to Table 21.

The content of active material and the stability were assessed at 40°C/75% RH in a closed HDPE bottle.

The results are given in Table 22.

It can be seen that the granules had a good homogeneity and no degradation of levocetirizine could be observed.

Moreover, the composition was substantialy free from the taste of levocetirizine when placed on the human tongue in dry form or when dispersed in water. Dissolution of a therapeutic dose of 5 mg of levocetirizine dihydrochloride in 50 ml of water took less than 2 minutes.

**Table 21 Composition example 13**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| β-cyclodextrin | 0.86 |
| Total | 100 |

**Table 22 Stability results example 13**

| Content in active drug (%) - 40°C/75%RH (n=5) | |
|---|---|
| T0 | T 1 Month |
| 99.9 ± 0.5% | 99.4 ± 0.6% |

As shown in experimental results of Table22, the obtained composition was stable and homogeneous. It had an acceptable taste and palatability.

### Example 14

Composition containing levocetirizine dihydrochloride were prepared by spray drying of an aqueous solution containing all materials. The pH of the solution was adjusted to 5.5 with HCI prior to spray drying.

The compositions is according to Table 23.

The content of active material and the stability were assessed at 40°C/75% RH in a closed HDPE bottle.

The results are given in Table 24.

It can be seen that the granules had a good homogeneity and no degradation of levocetirizine could be observed.

Moreover, the composition was substantialy free from the taste of levocetirizine when placed on the human tongue in dry form or when dispersed in water. Dissolution of a therapeutic dose of 5 mg of levocetirizine dihydrochloride in 50 ml of water took less than 2 minutes.

**Table 23 Composition example 14**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| β-cyclodextrin | 1.7 |
| Total | 100 |

**Table 24 Stability results example 14**

| Content in active drug (%) - 40°C/75%RH (n=5) | |
|---|---|
| T0 | T 1 Month |
| 99.3 ± 2.2% | 97.5 ± 0.6% |

As shown in experimental results of Table 24, the obtained composition was stable and homogeneous. It had an acceptable taste and palatability.

### Example 15

The composition containing levocetirizine dihydrochloride were prepared by fluid bed granulation process.
All materials except mannitol, anhydrous silicon dioxide and strawberry flavors were dissolved in water solution at pH 5.5 , granulated with mannitol and then dried. After drying, the granules were mixed with strawberry flavor, a part of the mannitol and anhydrous silicon dioxide.

**Table 25 Composition example 15**

| MATERIAL | QUANTITY % |
|---|---|
| Levocetirizine HCl | 0.3 |
| Trisodium citrate.2H2O | 2.3 |
| Citric acid (monohydrate) | 0.3 |
| hydroxypropylcellulose | 0.03 |
| Mannitol DC 300 (intra granular) | q.s. |
| Mannitol DC 300 (extra granular) | 5.0 |
| Sucralose | 0.2 |
| Strawberry flavor (extra granular) | 0.1 |
| Beta-cyclodextrin | 1.1 |
| Anhydrous silicon dioxide (extra granular) | 0.5 |

Mannitol (Pearlitol® DC300) is a granulated powder mannitol having an average particle size of about 250 - 350 µm.

Trisodium citrate (hydrated) and citric acid (hydrated) are used as buffering agent. Hydroxypropyl-cellulose (Klucel® EF) is a soluble binder. Sucralose is used as sweetner. B-cyclodextrine (Kleptose® 4PC) is used as taste masking agent. Strawberry flavor is used as flavoring agent, Anhydrous silicon dioxide (Aerosil ®200) is used as anti-caking agent
The obtained composition was stable and homogeneous. It had an acceptable taste and palatability. The obtained composition was an immediate release composition.

### Example 16

Composition containing levocetirizine dihydrochloride were prepared by spray drying of an aqueous solution containing all materials. The pH of the solution was adjusted to 5.5 with HCI prior to spray drying.

The compositions is according to Table 27.

The content of active material and the stability were assessed at 40°C/75% RH in a closed HDPE bottle.

The results are given in Table 28.

It can be seen that the granules had a good homogeneity and no degradation of levocetirizine could be observed.

Moreover, the composition was substantialy free from the taste of levocetirizine when placed on the human tongue in dry form or when dispersed in water.

Dissolution of a therapeutic dose of 5 mg of levocetirizine dihydrochloride in 50 ml of water took less than 2 minutes.

**Table 27 Composition example 16**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| β-cyclodextrin | 3.43 |
| Total | 100 |

**Table 28 Stability results example 16**

| Content in active drug (%) - 40°C/75%RH (n=5) | | |
|---|---|---|
| T0 | T 1 Month | T 3 Months |
| 99.7 ± 0.7% | 98.2 ± 0.6% | 98.7 ± 0.2% |

As shown in experimental results of Table 28, the obtained composition was stable and homogeneous. It had an acceptable taste and palatability.

### Example 17 (comparison exemple)

Composition containing levocetirizine dihydrochloride were prepared by spray drying of an aqueous solution containing all materials. The pH of the solution was adjusted to 4.0 with HCI prior to spray drying.

The compositions is according to Table 29.

The content of active material and the stability were assessed at 40°C/75% RH in a closed HDPE bottle.

The results are given in Table 30.

It can be seen that the granules had a good homogeneity but a trend for decrease in assay could be observed. This was a sign for levoceririzine degradation.

Dissolution of a therapeutic dose of 5 mg of levocetirizine dihydrochloride in 50 ml of water took less than 2 minutes.

**Table 29 Composition example 17**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| β-cyclodextrin | 2.57 |
| Total | 100 |

**Table 30 Stability results example 17**

| Content in active drug (%) - 40°C/75%RH (n=5) | | |
|---|---|---|
| T0 | T 1 Month | T 3 Months |
| 100.2 ± 0.7% | 95.5 ± 0.4% | 92.8 ± 1.2% |

As shown in experimental results of Table 30, the obtained composition was not stable.

### Example 18

Composition containing levocetirizine dihydrochloride were prepared by spray drying of an aqueous solution containing all materials. The pH of the solution was adjusted to 4.5 with HCI prior to spray drying.

The compositions is according to Table 31.

The content of active material and the stability were assessed at 40°C/75% RH in a closed HDPE bottle.

The results are given in Table 32.

It can be seen that the granules had a good homogeneity and no degradation of the levocetirizine could be observed.

Moreover, the composition was substantialy free from the taste of levocetirizine when placed on the human tongue in dry form or when dispersed in water.

The composition complied with the requirements.

**Table 31 Composition example 18**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| β-cyclodextrin | 3.43 |
| Total | 100 |

**Table 32 Stability results example 18**

| Content in active drug (%) - 40°C/75%RH (n=5) | | |
|---|---|---|
| T0 | T 1 Month | T 3 Months |
| 99.8 ± 0.8% | 100.3 ± 0.8% | 97.5 ± 1.2% |

As shown in experimental results of Table 32, the obtained composition was stable and homogeneous. It had an acceptable taste and palatability.

### Example 19

Composition containing levocetirizine dihydrochloride were prepared by spray drying of an aqueous solution containing all materials. The pH of the solution was adjusted to 7.0 with NaOH prior to spray drying.

The compositions is according to Table 33.

The content of active material and the stability were assessed at 40°C/75% RH in a closed HDPE bottle.

The results are given in Table 34.

It can be seen that the granules had a good homogeneity and no degradation of the levocetirizine could be observed.

Moreover, the composition was substantialy free from the taste of levocetirizine when placed on the human tongue in dry form or when dispersed in water.

The composition complied with the requirements.

**Table 33 Composition example 19**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| β-cyclodextrin | 3.43 |
| Total | 100 |

**Table 34 Stability results example 19**

| Content in active drug (%) - 40°C/75%RH (n=5) | | |
|---|---|---|
| T0 | T 1 Month | T 3 Months |
| 101.1 ± 0.9% | 100.7 ± 0.3% | 97.8 ± 0.3% |

As shown in experimental results of Table 34, the obtained composition was stable and homogeneous. It had an acceptable taste and palatability.

### Example 20

Composition containing levocetirizine dihydrochloride were prepared by spray drying of an aqueous solution containing all materials. The pH of the solution was adjusted to 6.0 with HCI prior to spray drying.

The compositions is according to Table 35.

The content of active material and the stability were assessed at 40°C/75% RH in a closed HDPE bottle.

The results are given in Table 36.

It can be seen that the granules had a good homogeneity and no degradation of the levocetirizine could be observed.

Moreover, the composition was substantialy free from the taste of levocetirizine when placed on the human tongue in dry form or when dispersed in water.

The composition complied with the requirements.

**Table 35 Composition example 20**

| MATERIAL | QUANTITY % |
|---|---|
| Levocetirizine HCl | 0.33 |
| Trisodium citrate.2H2O | 0.8 |
| Mannitol DC 400 | q.s. |
| Acesulfame K | 0.1 |
| Strawberry flavor | 0.1 |
| β-cyclodextrin | 3.43 |
| Total | 100 |

**Table 36 Stability results example 20**

| Content in active drug (%) - 40°C/75%RH (n=5) | | |
|---|---|---|
| T0 | T 1 Month | T 3 Months |
| 99.2 ± 1.6% | 100.4 ± 0.3% | 99.5 ± 0.5% |

As shown in experimental results of Table 36, the obtained composition was stable and homogeneous. It had an acceptable taste and palatability.

### Example 21

Composition containing levocetirizine dihydrochloride were prepared by fluid bed granulation process.

All materials except mannitol and strawberry flavors were dissolved in water and granulated with mannitol, and then dried. The pH of the solution was 5.5.

After drying, the granules were mixed with strawberry flavor.

The compositions is according to Table 37.

The content of active material and the stability were assessed at 40°C/75% RH in a closed HDPE bottle.

The results are given in Table 38.

It can be seen that the granules had a good homogeneity and no degradation of the levocetirizine could be observed.

Moreover, the composition was substantialy free from the taste of levocetirizine when placed on the human tongue in dry form or when dispersed in water. 1 g of dry syrup prepared in Example 21 was dissolved in 10 mL of water and taste was evaluated on a 3-point scale. No bitterness was detected. 1g of dry syrup prepared in Example 21 was directly placed in the mouth of the subject and taste was evaluated on a 3-point scale. No bitterness was detected.

The composition complied with the requirements.

**Table 37 Composition example 21**

| MATERIAL | QUANTITY % |
|---|---|
| Levocetirizine HCl | 0.31 |
| Sucralose | 0.20 |
| Mannitol DC 300 | 95.29 |
| Trisodium citrate.2H2O | 2.47 |
| Citric acid (anhydrous) | 0.33 |
| β-cyclodextrin (Kleptose standard) | 1.26 |
| Strawberry flavor | 0.10 |
| hydroxypropylcellulose | 0.03 |
| Total | 100.00 |

**Table 38 Stability results example 20**

| Content in active drug (%) - 40°C/75%RH | | | |
|---|---|---|---|
| | T0 (n=10) | T 1 Month (n=2) | T 3 Months |
| Assay | 98.0± 2.2% | 94.9 | 98.9 % |
| Impurities | Within ICHQ3b limits* | Within ICHQ3b limits | Within ICHQ3b limits |

| | | | |
|---|---|---|---|
| *ICH Q3b limits are set at 0.5% for individual specificed identified impurities and 0.2% for individual unspecified impurities. | | | |

| Sum of degradation products - 40°C/75%RH (n=2) | | |
|---|---|---|
| T0 | T 1 Month | T 3 Months |
| 0.0% | 0.0% | 0.1 % |

As shown in experimental results of Table 38, there was no degradation. The composition was stable.

### Example 22

Composition containing levocetirizine dihydrochloride were prepared by wet granulation process.

All materials except mannitol were dissolved in water and granulated with mannitol and dried. The pH of the solution was 5.0.
The compositions is according to Table 39.

The content of active material and the stability were assessed at 40°C/75% RH in an opened HDPE bottle.

The results are given in Table 40.

It can be seen that the granules had a good assay and no significant degradation of the levocetirizine could be observed.

Moreover, the composition was substantialy free from the taste of levocetirizine when placed on the human tongue in dry form or when dispersed in water. 1 g of dry syrup prepared in Example 22 was dissolved in 10 mL of water and taste was evaluated on a 3-point scale. No bitterness was detected.

The composition complied with the requirements.

**Table 39 Composition example 22**

| MATERIAL | QUANTITY % |
|---|---|
| Levocetirizine HCl | 0.31 |
| Sucralose | 0.10 |
| Mannitol DC 300 | q.s. |
| Trisodium citrate.2H2O | 2.17 |
| Citric acid (anhydrous) | 0.53 |
| β-cyclodextrin (Kleptose standard) | 1.26 |
| Total | 100.00 |

**Table 40 Stability results example 22**

| Content in active drug (%) - 40°C/75%RH (n=2) | | | |
|---|---|---|---|
| | T0 | T 1 Month * | T 3 Months * |
| Assay | 100% | 98.8 | 97.4% |
| Impurities | Within ICHQ3b limits** | Within ICHQ3b limits** | Within ICHQ3b limits** |

| | | | |
|---|---|---|---|
| **ICH Q3b (international guidance) limits are set at 0.5% for individual specificed identified impurities and 0.2% for individual unspecified impurities *relative to T0 | | | |

| Sun of degradation products - 40°C/75%RH (n=2) | | |
|---|---|---|
| T0 | T 1 Month | T 3 Months |
| 0.0% | 0.31% | 0.49% |

As shown in experimental results of Table 40, the obtained composition was stable and homogenous.

### Example 23

Composition containing levocetirizine dihydrochloride were prepared by wet granulation process.

All materials except mannitol were dissolved in water and granulated with mannitol and dried. The pH of the solution was 6.0.
The compositions is according to Table 41.

The content of active material and the stability were assessed at 40°C/75% RH in an opened HDPE bottle.

The results are given in Table 42.

It can be seen that the granules had a good assay and no significant degradation of the levocetirizine could be observed.

Moreover, the composition was substantialy free from the taste of levocetirizine when placed on the human tongue in dry form or when dispersed in water.

The composition complied with the requirements.

**Table 41 Composition example 23**

| MATERIAL | QUANTITY % |
|---|---|
| Levocetirizine HCl | 0.31 |
| Sucralose | 0.10 |
| Mannitol DC 300 | q.s. |
| Trisodium citrate.2H2O | 2.73 |
| Citric acid (anhydrous) | 0.17 |
| β-cyclodextrin (Kleptose standard) | 1.26 |
| Total | 100.00 |

**Table 42 Stability results example 23**

| Content in active drug (%) - 40°C/75%RH (n=2) | | | |
|---|---|---|---|
| | T0 | T 1 Month * | T 3 Months * |
| Assay | 100.0% | 101.1% | 100.8% |
| Impurities | Within ICHQ3b limits** | Within ICHQ3b limits** | Within ICHQ3b limits** |

| | | | |
|---|---|---|---|
| *relative to T0 **ICH Q3b limits are set at 0.5% for individual specificed identified impurities and 0.2% for individual unspecified impurities | | | |

| Sum of degradation ptoducts - 40°C/75%RH (n=2) | | |
|---|---|---|
| T0 | T 1 Month | T 3 Months |
| 0.0% | 0.14% | 0.46% |

As shown in experimental results of Table 42, the obtained composition was stable and homogenous.

### Example 24

Composition containing levocetirizine dihydrochloride were prepared by wet granulation process.

All materials except mannitol were dissolved in water and granulated with mannitol and dried. The pH of the solution was 5.5.
The compositions is according to Table 43.

The content of active material and the stability were assessed at 40°C/75% RH in an opened HDPE bottle.

The results are given in Table 44.

It can be seen that the granules had a good assay and no significant degradation of the levocetirizine could be observed.
Moreover, the composition was substantialy free from the taste of levocetirizine when placed on the human tongue in dry form or when dispersed in water. 1 g of dry syrup prepared in Example 25 was dissolved in 10 mL of water and taste was evaluated on a 3-point scale. No bitterness was detected.

The composition complied with the requirements.

**Table 43 Composition example 24**

| MATERIAL | QUANTITY % |
|---|---|
| Levocetirizine HCl | 0.31 |
| Sucralose | 0.10 |
| Mannitol DC 300 | q.s. |
| Trisodium citrate.2H2O | 2.47 |
| Citric acid (anhydrous) | 0.33 |
| β-cyclodextrin (Kleptose standard) | 1.26 |
| Total | 100.00 |

**Table 44 Stability results example 24**

| Content in active drug (%) - 40°C/75%RH (n=2) | | | |
|---|---|---|---|
| | T0 | T 1 Month * | T 3 Months * |
| Assay | 100.0% | 99.2% | 98.8% |
| Impurities | Within ICHQ3b limits** | Within ICHQ3b limits** | Within ICHQ3b limits** |

| | | | |
|---|---|---|---|
| *relative to T0 **ICH Q3b limits are set at 0.5% for individual specificed identified impurities and 0.2% for individual unspecified impurities | | | |

| Sum of degradation products - 40°C/75%RH (n=2) | | |
|---|---|---|
| T0 | T 1 Month | T 3 Months |
| 0% | 0.37% | 0.50% |

As shown in experimental results of Table 44, the obtained composition was stable and homogenous.

### Example 25 (comparaison example)

Composition containing levocetirizine dihydrochloride were prepared by wet granulation process.

All materials except mannitol were dissolved in water and granulated with mannitol and dried. The pH of the solution was not controlled with a buffer The compositions is according to Table 45.

The content of active material and the stability were assessed at 40°C/75% RH in an opened HDPE bottle.

The results are given in Table 46.

It can be seen that the granules showed a significant degradation of the levocetirizine.

**Table 45 Composition example 25**

| MATERIAL | QUANTITY % |
|---|---|
| Levocetirizine HCl | 0.31 |
| Sucralose | 0.10 |
| Mannitol DC 300 | q.s. |
| β-cyclodextrin (Kleptose standard) | 1.26 |
| Total | 100.00 |

**Table 46 Stability results example 25**

| Content in active drug (%) - 40°C/75%RH (n=2) | | | |
|---|---|---|---|
| | T0 | T 1 Month * | T 3 Months * |
| Assay | 100.0% | 74.4% | 74.3% |
| Impurities | Within ICHQ3b limits** | Out of ICHQ3b limits** | Out of ICHQ3b limits** |

| | | | |
|---|---|---|---|
| *relative to T0 **ICH Q3b limits are set at 0.5% for individual specificed identified impurities and 0.2% for individual unspecified impurities | | | |

| Sum of degradation products - 40°C/75%RH (n=2) | | |
|---|---|---|
| T0 | T 1 Month | T 3 Months |
| 0% | 25.6% | 23.8% |

This comparative example showed that a buffer system is needed to ensure appropriate stability of the composition.

### Example 26

Composition containing levocetirizine dihydrochloride were prepared by wet granulation process.

All materials except mannitol were dissolved in water and granulated with mannitol and dried. The pH of the solution was 5.5.
The compositions is according to Table 47.

The content of active material and the stability were assessed at 40°C/75% RH in an opened HDPE bottle.

The results are given in Table 48.

It can be seen that the granules had a good assay and no significant degradation of the levocetirizine could be observed.

Moreover, the composition was substantialy free from the taste of levocetirizine when placed on the human tongue in dry form or when dispersed in water.
1 g of dry syrup prepared in Example 26 was dissolved in 10 mL of water and taste was evaluated on a 3-point scale. No bitterness was detected.

The composition complied with the requirements.

**Table 47 Composition example 26**

| MATERIAL | QUANTITY % |
|---|---|
| Levocetirizine HCl | 0.31 |
| Sucralose | 0.10 |
| Mannitol DC 300 | q.s. |
| Trisodium citrate.2H2O | 4.12 |
| Citric acid (anhydrous) | 0.56 |
| β-cyclodextrin (Kleptose standard) | 1.26 |
| Total | 100.00 |

**Table 48 Stability results example 26**

| Content in active drug (%) - 40°C/75%RH (n=2) | | | |
|---|---|---|---|
| | T0 | T 1 Month * | T 3 Months * |
| Assay | 100.0% | 99.2% | 98.6% |
| Impurities | Within ICHQ3b limits** | Within ICHQ3b limits** | Within ICHQ3b limits** |

| | | | |
|---|---|---|---|
| *relative to T0 **ICH Q3b limits are set at 0.5% for individual specificed identified impurities and 0.2% for individual unspecified impurities *relative to T0 | | | |

| Sum of degradation products - 40°C/75%RH (n=2) Open dish | | |
|---|---|---|
| T0 | T 1 Month | T 3 Months |
| 0% | 0.24% | 0.51% |

As shown in experimental results of Table 48, the obtained composition was stable and homogenous.

### Example 27 Levocetirizine dry syrup composition

The dry syrup was prepared by wet granulation process according to the invention with the following composition (Table 49).

**Table 49 Composition example 27**

| Material | % | Part |
|---|---|---|
| Levocetirizine HCl | 0.325 | Coating 2 |
| Betacyclodextrin | 2.667 | coating 2 |
| Acesulfame K | 0.090 | coating 2 |
| Trisodium citrate.2H2O | 0.415 | coating 1 |
| Trisodium citrate.2H2O | 0.415 | coating 2 |
| Hydroxypropylmethyl cellulose HPMC 5cp | 5.767 | Coating 1 |
| Mannitol DC 400 | 90.098 | Core |
| Strawberry flavor | 0.224 | Coating 2 |
| Total | 100.0 | |

Mannitol (Mannitol DC400) is a granulated powder mannitol having an average particle size of about 300 - 400 µm. Trisodium citrate and HCl are used as buffering agent. Hydroxypropylmethyl cellulose(HPMC 5cp) is used as polymer to separate the second coating from the core. Acesulfame K (acesulfame potassium) is used as sweetener.
Beta- cyclodextrin (Kleptose® DC) is used as taste masking agent.
Strawberry flavor is used as flavoring agent.

The granules were prepared by spraying an aqueous solution of the materials (hydroxypropylmethyl cellulose and sodium citrate) from the first coating onto the mannitol core particles.

After drying, an aqueous solution containing the materials (levocetirizine, beta-cyclodextrin, flavor, Na citrate, Acesulfame K) from the second coating was sprayed onto the intermediate granules.
Both spray solutions were buffered at pH 5.5, by using HCI to adjust pH. A fluid bed coater was used to the purpose.

The compositions were placed at 40°C - 75 % relative humidity (RH) during 2 weeks. Table 50 gives the results of this stability study.

**Table 50 : stability results example 27**

| Time | Content of levocetirizine (40°C/75%RH) |
|---|---|
| week | |
| 0 | 98,22 ± 0,98 |
| 1 | 98,81 ± 0,35 |
| 2 | 95,22 ± 1,41 |

The results show the composition was homogeneous and stable. The pharmaceutical composition was completely soluble within less than 2 minutes in a glass containing about 50 ml of water at room temperature.

The composition complied with the requirements.
1 g of dry syrup prepared in Example 27 was directly placed in the mouth of the subject and taste was evaluated on a 3-point scale. No bitterness was detected.

### Example 28 : Levocetirizine dry syrup composition

The dry syrup was prepared by wet granulation process with the following composition (Table 51).

**Table 51 Composition example 28**

| Material | % | Part |
|---|---|---|
| Levocetirizine HCl | 0.484 | coating 2 |
| Betacyclodextrin | 3.968 | coating 1 |
| Acesulfame K | 0.133 | coating 2 |
| Trisodium citrate | 0.616 | coating 1 |
| Trisodium citrate | 0.616 | coating 2 |
| Mannitol DC 400 | 93.846 | Core |
| Strawberry flavor | 0.336 | extra-granular phase |
| Total | 100.0 | |

Mannitol (Mannitol DC400) is a granulated powder mannitol having an average particle size of about 300 - 400 µm. Sodium citrate and HCl are used as buffering agent. Acesulfame K (acesulfame potassium) is used as sweetner. Beta-cyclodextrin (Kleptose® DC) is used as taste masking agent. Strawberry flavor is used as flavoring agent.

The granules were prepared by spraying an aqueous solution of the materials from the first coating onto the mannitol core particles.

After drying, a solution containing the materials from the second coating was sprayed onto the intermediate granules.

Both spray solutions were buffered at pH 5.5, by using HCl to adjust pH.

A fluid bed coater was used to the purpose.
Strawberry flavor was added to the granules by physical mix.

The compositions were placed at 40°C - 75 % relative humidity (RH) during 4 weeks. Table 52 gives the results of this stability study.

**Table 52 stability results example 28**

| Time | Content in levocetirizine |
|---|---|
| week | |
| 0 | 99,04 ± 3,28 |
| 2 | 98,38 ± 1,23 |
| 4 | 104,55 ± 2,29 |

The results show the composition was homogeneous and stable. The pharmaceutical composition was completely soluble within less than 2 minutes in a glass containing about 50 ml of water at room temperature.

The composition complied with the requirements.
1 g of dry syrup prepared in Example 28 was directly placed in the mouth of the subject and taste was evaluated on a 3-point scale. No bitterness was detected.

### Example 29 :

Levocetirizine dry syrup composition
The dry syrup was prepared by wet granulation process with the following composition

**Table 53 Composition example 29**

| Material | % | Part |
|---|---|---|
| Levocetirizine HCl | 0.3 | Coating 2 |
| Betacyclodextrin | 1.1 | coating 2 |
| Sucralose | 0.090 | coating 2 |
| Trisodium citrate.2H2O | 2.2 | coating 2 |
| Citric acid (monohydrate) | 0.3 | coating 2 |
| hydroxypropylcellulose | 0.03 | coating 2 |
| Hydroxypropylmethyl cellulose | 5 | Coating 1 |
| Mannitol DC 300 | q.s. | Core |
| Strawberry flavor | 0.224 | Extra Granular phase |
| Mannitol DC 300 | 2.5 | Extra Granular phase |
| Colloidal anhydrous silica | 0.5 | Extra Granular phase |

Mannitol (Mannitol DC300) is a granulated powder mannitol having an average particle size of about 250 - 350 µm. Trisodium citrate and citric acid are used as buffering agent. Hydroxypropylmethyl cellulose(HPMC 5cp) is used as polymer to separate the second coating from the core. Sucralose is used as sweetener. Beta- cyclodextrin (Kleptose® 4 pc) is used as taste masking agent.
Strawberry flavor is used as flavoring agent. Colloidal anhydrous silica (aerosil 200) is used as flow enhancer/antisticking agent. Hydroxypropylcellulose (Klucel EF) is used as binder.

The granules were prepared by spraying an aqueous solution of the materials (hydroxypropylmethyl cellulose) from the first coating onto the mannitol core particles.

After drying, an aqueous solution containing the materials (levocetirizine, beta-cyclodextrin, Na citrate, citric acid and sucralose) from the second coating was sprayed onto the intermediate granules.

A fluid bed coater was used to the purpose.

The obtained granules were subsequently mixed with the extra-granular materials.

The obtained composition was homogeneous and stable. No significant degradation of the levocetirizine could be observed. 1 g of dry syrup prepared in Example 29 was directly placed in the mouth of the subject and taste was evaluated on a 3-point scale. No bitterness was detected.

The pharmaceutical composition was completely soluble within less than 2 minutes in a glass containing about 50 ml of water at room temperature.

The composition complied with the requirements.

### Example 30 : Levocetirizine orodispersible composition

The granule containing levocetirizine dihydrochloride were prepared by fluid bed granulation process.

All materials except mannitol, were dissolved in water, granulated with mannitol and dried. After drying, the granules were mixed with extragranular excipients : mannitol, sodium starch glycolate, magnesium stearate and anhydrous silicon dioxide and then compressed on a tablet press to obtain an oro-dispersible tablet.

**Table 54 Composition example 30**

| MATERIAL | QUANTITY % |
|---|---|
| Intra-granular phase | |
| Levocetirizine HCl | 0.15 |
| Trisodium citrate.2H2O | 1.25 |
| Citric acid (monohydrate) | 0.2 |
| hydroxypropylcellulose | 0.02 |
| Mannitol DC 300 | 48.87 |
| Sucralose | 0.05 |
| Beta-cyclodextrin | 0.6 |

| Extra-granular phase | |
|---|---|
| Mannitol DC300 | 45 |
| Sodium starch glycolate | 3 |
| Magnesium stearate | 1 |
| Anhydrous silicon dioxide | 1.0 |
| Total | 100 |

Mannitol (Pearlitol ®DC300) is a granulated powder mannitol having an average particle size of about 250 - 350 µm. it is used as granule carrier and as diluent.

Sodium citrate (hydrated) and citric acid (hydrated) are used as buffering agent. Hydroxypropyl-cellulose (Klucel ®EF) is a soluble binder. Sucralose is used as sweetner. B-cyclodextrine (Kleptose ®4PC) is used as taste masking agent. Anhydrous silicon dioxide (Aerosil ®200) is used as anti-caking agent. Magnesium Stearate is used as lubricant. Sodium starch glycolate is used as desintegrant.

The obtained composition was stable and homogeneous. It had an acceptable taste and palatability. The obtained composition was dissolved within less than 3 min, as per pharmacopeia test for disintegration of tablets and capsules.
1 g of oro-dispersible composition prepared in Example 30 was dissolved in 10 mL of water and taste was evaluated on a 3-point scale. No bitterness was detected.
It was an oro-dispersible tablet.

### Example 31 : levocetirizine orodispersible tablet

The granule containing levocetirizine were prepared by fluid bed granulation process.

All materials except mannitol, were dissolved in water, granulated with mannitol and dried. After drying, the granules were mixed with extragranular excipients : Pearlitol Flash® (combo excipient of mannitol and starch), sodium starch glycolate, magnesium stearate and anhydrous silicon dioxide and then compressed on a tablet press to obtain an oro-dispersible tablet.

**Table 55 composition example 31**

| MATERIAL | QUANTITY % |
|---|---|
| Intra-granular phase | |
| Levocetirizine HCl | 0.22 |
| Trisodium citrate.2H2O | 1.9 |
| Citric acid (monohvdrate) | 0.3 |
| hydroxypropylcellulose | 0.03 |
| Mannitol DC 300 | 73.3 |
| Sucralose | 0.08 |
| Beta-cyclodextrin | 0.9 |

| Extra-granular phase | |
|---|---|
| Pearlitol Flash® | 18.27 |
| Sodium starch glycolate | 3 |
| Magnesium stearate | 1 |
| Anhydrous silicon dioxide | 1.0 |
| Total | 100 |

Mannitol (Pearlitol ®DC300) is a granulated powder mannitol having an average particle size of about 250 - 350 µm. it is used as granule carrier and as diluent.

Sodium citrate (hydrated) and citric acid (hydrated) are used as buffering agent. Hydroxypropyl-cellulose (Klucel ®EF) is a soluble binder. Sucralose is used as sweetner. B-cyclodextrine (Kleptose ®4PC) is used as taste masking agent. Anhydrous silicon dioxide (Aerosil ®200) is used as anti-caking agent. Magnesium Stearate is used as lubricant. Sodium starch glycolate is used as desintegrant.

The obtained composition was stable and homogeneous. It had an acceptable taste and palatability. 1 g of oro-dispersible composition prepared in Example 31 was dissolved in 10 mL of water and taste was evaluated on a 3-point scale. No bitterness was detected.

The obtained composition was dissolved within less than 3 min, as per pharmacopeia test for disintegration of tablets and capsules. It was an oro-dispersible tablet.

In all examples, levocetirizine dihydrochloride can be substituted by any other levocetirizine salt or by the free base, as this active material is solubilized in the spray solution prior to granulation. Only weight ratios should be adjusted accordingly.

## Claims

1. A pharmaceutical composition, in a solid form, allowing an oral administration of an unit dose ranging from 0.50 mg to 25.00 mg of levocetirizine dihydrochloride as active ingredient, and containing active granules comprising
- the active ingredient,
- a polyol fraction comprising one or more solid water soluble polyols having a molecular weight below 950 g/ mol, with a molar ratio between the polyol fraction and the active ingredient higher than 50, at least one solid water soluble polyol being mannitol, and
- a buffering system, which contributes to maintain the pH of the whole pharmaceutical composition between 4.0 and 7.0 when the pharmaceutical composition containing 5 mg of active ingredient is dissolved in 100 ml of water.

2. The composition according to claim 1, wherein the composition comprises 0.1 to 2.0 % per weight of active compound with respect to the total weight of the composition.

3. The composition according to claim 1 or 2 wherein the composition comprises active granules in an amount of 25 to 100 % with respect to the total weight of the pharmaceutical composition.

4. The composition according to any of claims 1-3 wherein the active granule comprises a buffering system which contributes to maintain the pH of the whole pharmaceutical composition in a range of pH 5.5 ± 0.5 when the pharmaceutical composition containing 5 mg of active ingredient is dissolved in 100 ml of water.

5. The composition according to any of claims 1- 4 wherein the active granule is buffered at a concentration ranging from 1.10⁻⁴ mol/l to 1.10⁻² mol/l when the pharmaceutical composition containing 5 mg of active ingredient is dissolved in 100 ml of water.

6. The composition according to any of the preceding claims wherein the buffering system is selected among pharmaceutical acceptable salts of phosphate, citrate, tartrate, acetate, fumarate, gluconate, used as such or in combination with their respective related acid, or mixtures thereof.

7. The composition according to any of preceding claims wherein the active granules contain at least a water soluble excipient selected from water soluble polymer, cyclodextrin or mixtures thereof.

8. The composition according to claim 7 wherein the cyclodextrin is selected among alpha cyclodextrin, β-cydodextrin, hydroxypropyl-β-cyclodextrin, methyl-β-cyclodextrin sulfo alkyl ether cyclodextrin, gamma-cyclodextrin or mixture thereof.

9. The pharmaceutical composition according to claim 1, wherein it contains active granules in an amount of 25 to 100% with respect of the total weight of the composition, these active granules comprising
- 0.1 to 2 % of levocetirizine as active ingredient, with respect of the total weight of the composition,
- at least 50 % of a solid water soluble polyol having a molecular weight below 950 g/mol, with respect of the total weight of the composition, and
- a buffering system at a concentration ranging from 1.10⁻⁴ mol/l to 1.10⁻² mol/l of buffering system, when the pharmaceutical composition containing 5 mg of active ingredient is dissolved in 100ml of water, which contributes to maintain the pH of the pharmaceutical composition between 4.0 and 7.0.

10. The pharmaceutical composition according to claim 1, wherein it contains active granules in an amount of 50 to 100% with respect of the total weight of the composition, these active granules comprising
- 0.1 to 1.0 % of levocetirizine as active ingredient, with respect of the total weight of the composition,
- at least 70 % of a solid water soluble polyol having a molecular weight below 350 g/mol, with respect of the total weight of the composition,
- a buffering system at a concentration ranging from 2.10⁻⁴ mol/l to 5.10⁻³ mol/l of buffering system, when the pharmaceutical composition containing 5 mg of active ingredient is dissolved in 100ml of water,which contributes to maintain the pH of the pharmaceutical composition in a range of pH 5.5 ± 0.5,
- a water soluble excipient selected among a cyclodextrin, the molar ratio between cyclodextrin and the active ingredient being between 0 to 4; a water soluble polymer, the weight ratio between the polymer and the granule being between 0 to 2 %; or mixture thereof.

11. The pharmaceutical composition according to any of the preceding claims, wherein the active granules comprise
- a core which comprises at least a solid water soluble polyol having a molecular weight below 950 g/ mol;
- a first coating applied onto the core, and comprising at least an excipient;
- a second coating applied onto the first coating, and comprising the active ingredient and a buffering system.

12. The pharmaceutical composition according to claim 11, wherein the excipient of the first coating is a water soluble excipient selected from water soluble polymer, polymer dispersion, cyclodextrin, and mixtures thereof.

13. The pharmaceutical composition according to claims 11 or 12, wherein the second coating comprises 0.1 to 4 % per weight of active ingredient with respect to the total weight of the pharmaceutical composition.

14. The pharmaceutical composition according to any of the claims 11 to 13, wherein the second coating comprises a buffering system which contribute to maintain the pH of the second coating and of the whole pharmaceutical composition between 4.0 and 7.0, when the pharmaceutical composition containing 5 mg of active ingredient is dissolved in 100ml of water.

15. The pharmaceutical composition according to any of the claims 11 to 14,
wherein the second coating comprises cyclodextrin.

16. A pharmaceutical composition according to claim 11, wherein the composition comprises
- a core which comprises at least mannitol;
- a first coating applied onto the core, and comprising at least cyclodextrin; and
- a second coating applied onto the first coating, and comprising levocetirizine, a buffering system and at least a water soluble excipient.

17. The pharmaceutical composition according to claim 16, wherein the composition allows an oral administration of an unit dose ranging from 1.00 mg to 10.00 mg of levocetirizine, as active ingredient, and comprises
- a core which comprises at least mannitol;
- a first coating applied onto the core, and comprising at least beta-cyclodextrin, the molar ratio between cyclodextrin and levocetirizine being comprised between 0 and 3, and comprising also a buffering system which contributes to maintain the pH between 4.5 and 6.5, when the pharmaceutical composition containing 5 mg of active ingredient is dissolved in 100ml of water;
- a second coating applied onto the first coating, and comprising levocetirizine at least a water soluble excipient, and a buffering system which contributes to maintain the pH between 4.5 and 6.5, when the pharmaceutical composition containing 5 mg of active ingredient is dissolved in 100ml of water; and
- optionally, a final water soluble coating applied onto the second coating.

18. A pharmaceutical composition according to claim 11, wherein the composition comprises
- a core which comprises at least mannitol;
- a first coating applied onto the core, and comprising at least hydroxypropyl methylcellulose or hydroxypropyl cellulose; and
- a second coating applied onto the first coating, and comprising levocetirizine, at least cyclodextrin and comprising also a buffering system which contributes to maintain the pH between 4.5 and 6.5, when the pharmaceutical composition containing 5 mg of active ingredient is dissolved in 100ml of water.

19. The pharmaceutical composition according to any of the preceding claims,
wherein the composition is in the form of a tablet, orally disintegrating tablet and a capsule.

20. The pharmaceutical composition according to claims 1 to 18, wherein the composition is in the form of dry syrup or granulate, that can be filled in a sachet or any appropriate dosing device.

## Patentansprüche

1. Pharmazeutische Zusammensetzung in einer festen Form, die eine orale Verabreichung einer Einheitsdosis im Bereich von 0,50 mg bis 25,00 mg Levocetirizindihydrochlorid als Wirkstoff erlaubt und ein aktives Granulat enthält, welches Folgendes umfasst:
- den Wirkstoff,
- eine Polyolfraktion mit einem oder mehreren festen wasserlöslichen Polyolen mit einem Molekulargewicht von unter 950 g/mol, mit einem Molverhältnis von Polyolfraktion zu Wirkstoff von über 50, wobei es sich bei mindestens einem festen wasserlöslichen Polyol um Mannitol handelt, und
- ein Puffersystem, welches dazu beiträgt, den pH-Wert der gesamten pharmazeutischen Zusammensetzung zwischen 4,0 und 7,0 zu halten, wenn man die 5 mg Wirkstoff enthaltende pharmazeutische Zusammensetzung in 100 ml Wasser löst.

2. Zusammensetzung nach Anspruch 1, wobei die Zusammensetzung 0,1 bis 2,0 Gew.-% Wirkstoff, bezogen auf das Gesamtgewicht der Zusammensetzung, umfasst.

3. Zusammensetzung nach Anspruch 1 oder 2, wobei die Zusammensetzung aktives Granulat in einer Menge von 25 bis 100 %, bezogen auf das Gesamtgewicht der pharmazeutischen Zusammensetzung, umfasst.

4. Zusammensetzung nach einem der Ansprüche 1-3, wobei das aktive Granulat ein Puffersystem umfasst, welches dazu beiträgt, den pH-Wert der gesamten pharmazeutischen Zusammensetzung in einem Bereich von pH 5,5 ± 0,5 zu halten, wenn man die 5 mg Wirkstoff enthaltende pharmazeutische Zusammensetzung in 100 ml Wasser löst.

5. Zusammensetzung nach einem der Ansprüche 1-4, wobei das aktive Granulat bei einer Konzentration im Bereich von 1 x 10⁻⁴ mol/l bis 1 x 10⁻² mol/l gepuffert wird, wenn man die 5 mg Wirkstoff enthaltende pharmazeutische Zusammensetzung in 100 ml Wasser löst.

6. Zusammensetzung nach einem der vorhergehenden Ansprüche, wobei das Puffersystem aus pharmazeutisch unbedenklichen Salzen von Phosphat, Citrat, Tartrat, Acetat, Fumarat, Gluconat, verwendet als solche oder in Kombination mit ihrer jeweiligen verwandten Säure, oder Mischungen davon ausgewählt ist.

7. Zusammensetzung nach einem der vorhergehenden Ansprüche, wobei das aktive Granulat mindestens einen wasserlöslichen Exzipienten ausgewählt aus einem wasserlöslichen Polymer, Cyclodextrin oder Mischungen davon enthält.

8. Zusammensetzung nach Anspruch 7, wobei das Cyclodextrin ausgewählt ist aus alpha-Cyclodextrin, β-Cyclodextrin, Hydroxypropyl-β-cyclodextrin, Methyl-β-cyclodextrin, Sulfoalkylethercyclodextrin, gamma-Cyclodextrin oder einer Mischung davon.

9. Pharmazeutische Zusammensetzung nach Anspruch 1, die aktives Granulat in einer Menge von 25 bis 100%, bezogen auf das Gesamtgewicht der Zusammensetzung, enthält, wobei dieses aktive Granulat Folgendes umfasst:
- 0,1 bis 2 % Levocetirizin als Wirkstoff, bezogen auf das Gesamtgewicht der Zusammensetzung,
- mindestens 50 % eines festen wasserlöslichen Polyols mit einem Molekulargewicht von unter 950 g/mol, bezogen auf das Gesamtgewicht der Zusammensetzung, und
- ein Puffersystem bei einer Konzentration im Bereich von 1 x 10⁻⁴ mol/l bis 1 x 10⁻² mol/l Puffersystem, wenn man die 5 mg Wirkstoff enthaltende pharmazeutische Zusammensetzung in 100 ml Wasser löst, welches dazu beiträgt, den pH-Wert der pharmazeutischen Zusammensetzung zwischen 4,0 und 7,0 zu halten.

10. Pharmazeutische Zusammensetzung nach Anspruch 1, die aktives Granulat in einer Menge von 50 bis 100%, bezogen auf das Gesamtgewicht der Zusammensetzung, enthält, wobei dieses aktive Granulat Folgendes umfasst:
- 0,1 bis 1,0 % Levocetirizin als Wirkstoff, bezogen auf das Gesamtgewicht der Zusammensetzung,
- mindestens 70 % eines festen wasserlöslichen Polyols mit einem Molekulargewicht von unter 350 g/mol, bezogen auf das Gesamtgewicht der Zusammensetzung,
- ein Puffersystem bei einer Konzentration im Bereich von 2 x 10⁻⁴ mol/l bis 5 x 10⁻³ mol/l Puffersystem, wenn man die 5 mg Wirkstoff enthaltende pharmazeutische Zusammensetzung in 100 ml Wasser löst, welches dazu beiträgt, den pH-Wert der pharmazeutischen Zusammensetzung in einem Bereich von pH 5,5 ± 0,5 zu halten,
- einen wasserlöslichen Exzipienten ausgewählt aus einem Cyclodextrin, wobei das Molverhältnis von Cyclodextrin zu Wirkstoff zwischen 0 und 4 liegt; einem wasserlöslichen Polymer, wobei das Gewichtsverhältnis von Polymer zu Granulat zwischen 0 und 2 % liegt; oder einer Mischung davon.

11. Pharmazeutische Zusammensetzung nach einem der vorhergehenden Ansprüche, wobei das aktive Granulat Folgendes umfasst:
- einen Kern, welcher mindestens einen festen wasserlöslichen Polyol mit einem Molekulargewicht von unter 950 g/mol umfasst;
- einen auf dem Kern aufgetragenen ersten Überzug, der mindestens einen Exzipienten umfasst;
- einen auf dem ersten Überzug aufgetragenen zweiten Überzug, der den Wirkstoff und ein Puffersystem umfasst.

12. Pharmazeutische Zusammensetzung nach Anspruch 11, wobei es sich bei dem Exzipienten des ersten Überzugs um einen wasserlöslichen Exzipienten ausgewählt aus einem wasserlöslichen Polymer, einer Polymerdispersion, Cyclodextrin und Mischungen davon handelt.

13. Pharmazeutische Zusammensetzung nach Anspruch 11 oder 12, wobei der zweite Überzug 0,1 bis 4 Gew.-% Wirkstoff, bezogen auf das Gesamtgewicht der pharmazeutischen Zusammensetzung, umfasst.

14. Pharmazeutische Zusammensetzung nach einem der Ansprüche 11 bis 13, wobei der zweite Überzug ein Puffersystem umfasst, welches dazu beiträgt, den pH-Wert des zweiten Überzugs der gesamten pharmazeutischen Zusammensetzung zwischen 4,0 and 7,0 zu halten, wenn man die 5 mg Wirkstoff enthaltende pharmazeutische Zusammensetzung in 100 ml Wasser löst.

15. Pharmazeutische Zusammensetzung nach einem der Ansprüche 11 bis 14, wobei der zweite Überzug Cyclodextrin umfasst.

16. Pharmazeutische Zusammensetzung nach Anspruch 11, wobei die Zusammensetzung Folgendes umfasst:
- einen Kern, welcher mindestens Mannitol umfasst;
- einen auf dem Kern aufgetragenen ersten Überzug, der mindestens Cyclodextrin umfasst; und
- einen auf dem ersten Überzug aufgetragenen zweiten Überzug, der Levocetirizin, ein Puffersystem und mindestens einen wasserlöslichen Exzipienten umfasst.

17. Pharmazeutische Zusammensetzung nach Anspruch 16, wobei die Zusammensetzung eine orale Verabreichung einer Einheitsdosis im Bereich von 1,00 mg bis 10,00 mg Levocetirizin als Wirkstoff erlaubt und Folgendes umfasst:
- einen Kern, welcher mindestens Mannitol umfasst;
- einen auf dem Kern aufgetragenen ersten Überzug, der mindestens beta-Cyclodextrin umfasst, wobei das Molverhältnis von Cyclodextrin zu Levocetirizin zwischen 0 und 3 liegt, und welcher außerdem ein Puffersystem umfasst, welches dazu beiträgt, den pH-Wert zwischen 4,5 and 6,5 zu halten, wenn man die 5 mg Wirkstoff enthaltende pharmazeutische Zusammensetzung in 100 ml Wasser löst;
- einen auf dem ersten Überzug aufgetragenen zweiten Überzug, der Levocetirizin, mindestens einen wasserlöslichen Exzipienten und ein Puffersystem umfasst, welches dazu beiträgt, den pH-Wert zwischen 4,5 and 6,5 zu halten, wenn man die 5 mg Wirkstoff enthaltende pharmazeutische Zusammensetzung in 100 ml Wasser löst; und
- gegebenenfalls einen auf dem zweiten Überzug aufgetragenen wasserlöslichen Endüberzug.

18. Pharmazeutische Zusammensetzung nach Anspruch 11, wobei die Zusammensetzung Folgendes umfasst:
- einen Kern, welcher mindestens Mannitol umfasst;
- einen auf dem Kern aufgetragenen ersten Überzug, der mindestens Hydroxypropylmethylcellulose oder Hydroxypropylcellulose umfasst; und
- einen auf dem ersten Überzug aufgetragenen zweiten Überzug, der Levocetirizin, mindestens Cyclodextrin und außerdem ein Puffersystem umfasst, welches dazu beiträgt, den pH-Wert zwischen 4,5 and 6,5 zu halten, wenn man die 5 mg Wirkstoff enthaltende pharmazeutische Zusammensetzung in 100 ml Wasser löst.

19. Pharmazeutische Zusammensetzung nach einem der vorhergehenden Ansprüche, wobei die Zusammensetzung in Form einer Tablette, einer im Mund zerfallenden Tablette und einer Kapsel vorliegt.

20. Pharmazeutische Zusammensetzung nach den Ansprüchen 1 bis 18, wobei die Zusammensetzung in Form von Trockensirup oder Granulat, der/das in einen Beutel oder eine beliebige geeignete Dosiervorrichtung abgefüllt werden kann, vorliegt.

## Revendications

1. Composition pharmaceutique, sous une forme solide, permettant une administration orale d'une dose unitaire dans la plage de 0,50 mg à 25,00 mg de dichlorhydrate de lévocétirizine en tant que substance active, et contenant des granules actifs comprenant
- la substance active,
- une fraction de polyols comprenant un ou plusieurs polyols hydrosolubles solides ayant un poids moléculaire inférieur à 950 g/mol, avec un rapport molaire entre la fraction de polyols et la substance active supérieur à 50, au moins un polyol hydrosoluble solide étant le mannitol, et
- un système tampon, qui contribue à maintenir le pH de la composition pharmaceutique entière entre 4,0 et 7,0 lorsque la composition pharmaceutique contenant 5 mg de substance active est dissoute dans 100 ml de eau.

2. Composition selon la revendication 1, la composition comprenant 0,1 à 2,0 % en poids de composé actif par rapport au poids total de la composition.

3. Composition selon la revendication 1 ou 2, la composition comprenant des granules actifs dans une quantité de 25 à 100 % par rapport au poids total de la composition pharmaceutique.

4. Composition selon l'une quelconque des revendications 1 à 3 dans laquelle le granule actif comprend un système tampon qui contribue à maintenir le pH de la composition pharmaceutique entière dans une plage de pH 5,5 ± 0,5 lorsque la composition pharmaceutique contenant 5 mg de substance active est dissoute dans 100 ml d'eau.

5. Composition selon l'une quelconque des revendications 1 à 4 dans laquelle le granule actif est tamponné à une concentration dans la plage de 1.10⁻⁴ mol/l à 1.10⁻² mol/l lorsque la composition pharmaceutique contenant 5 mg de substance active est dissoute dans 100 ml d'eau.

6. Composition selon l'une quelconque des revendications précédentes dans laquelle le système tampon est choisi parmi des sels pharmaceutiques acceptables de phosphate, citrate, tartrate, acétate, fumarate, gluconate, utilisés tels quels ou en combinaison avec leur acide associé respectif, ou des mélanges de ceux-ci.

7. Composition selon l'une quelconque des revendications précédentes dans laquelle les granules actifs contiennent au moins un excipient hydrosoluble choisi parmi un polymère hydrosoluble, une cyclodextrine ou des mélanges de ceux-ci.

8. Composition selon la revendication 7 dans laquelle la cyclodextrine est choisie parmi l'alpha-cyclodextrine, la β-cyclodextrine, l'hydroxypropyl-β-cyclodextrine, la méthyl-β-cyclodextrine, l'(éther sulfoalkylique)cyclodextrine, la gamma-cyclodextrine ou un mélange de celles-ci.

9. Composition pharmaceutique selon la revendication 1, qui contient des granules actifs dans une quantité de 25 à 100 % par rapport au poids total de la composition, ces granules actifs comprenant
- 0,1 à 2 % de lévocétirizine en tant que substance active, par rapport au poids total de la composition,
- au moins 50 % d'un polyol hydrosoluble solide ayant un poids moléculaire inférieur à 950 g/mol, par rapport au poids total de la composition, et
- un système tampon à une concentration dans la plage de 1.10⁻⁴ mol/l à 1.10⁻² mol/l de système tampon, lorsque la composition pharmaceutique contenant 5 mg de substance active est dissoute dans 100 ml d'eau, qui contribue à maintenir le pH de la composition pharmaceutique entre 4,0 et 7,0.

10. Composition pharmaceutique selon la revendication 1, qui contient des granules actifs dans une quantité de 50 à 100 % par rapport au poids total de la composition, ces granules actifs comprenant
- 0,1 à 1,0 % de lévocétirizine en tant que substance active, par rapport au poids total de la composition,
- au moins 70 % d'un polyol hydrosoluble solide ayant un poids moléculaire inférieur à 350 g/mol, par rapport au poids total de la composition,
- un système tampon à une concentration dans la plage de 2.10⁻⁴ mol/l à 5.10⁻³ mol/l de système tampon, lorsque la composition pharmaceutique contenant 5 mg de substance active est dissoute dans 100 ml d'eau, qui contribue à maintenir le pH de la composition pharmaceutique dans une plage de pH 5,5 ± 0,5,
- un excipient hydrosoluble choisi parmi une cyclodextrine, le rapport molaire entre la cyclodextrine et la substance active étant compris entre 0 et 4 ; un polymère hydrosoluble, le rapport en poids entre le polymère et le granule étant compris entre 0 et 2 % ; ou un mélange de ceux-ci.

11. Composition pharmaceutique selon l'une quelconque des revendications précédentes, dans laquelle les granules actifs comprennent
- un noyau qui comprend au moins un polyol hydrosoluble solide ayant un poids moléculaire inférieur à 950 g/mol ;
- un premier enrobage appliqué sur le noyau, et comprenant au moins un excipient ;
- un deuxième enrobage appliqué sur le premier enrobage, et comprenant la substance active et un système tampon.

12. Composition pharmaceutique selon la revendication 11, dans laquelle l'excipient du premier enrobage est un excipient hydrosoluble choisi parmi un polymère hydrosoluble, une dispersion de polymère, une cyclodextrine, et des mélanges de ceux-ci.

13. Composition pharmaceutique selon les revendications 11 ou 12, dans laquelle le deuxième enrobage comprend 0,1 à 4 % en poids de substance active par rapport au poids total de la composition pharmaceutique.

14. Composition pharmaceutique selon l'une quelconque des revendications 11 à 13, dans laquelle le deuxième enrobage comprend un système tampon qui contribue à maintenir le pH du deuxième enrobage et de la composition pharmaceutique entière entre 4,0 et 7,0, lorsque la composition pharmaceutique contenant 5 mg de substance active est dissoute dans 100 ml d'eau.

15. Composition pharmaceutique selon l'une quelconque des revendications 11 à 14, dans laquelle le deuxième enrobage comprend de la cyclodextrine.

16. Composition pharmaceutique selon la revendication 11, la composition comprenant
- un noyau qui comprend au moins du mannitol ;
- un premier enrobage appliqué sur le noyau, et comprenant au moins de la cyclodextrine ; et
- un deuxième enrobage appliqué sur le premier enrobage, et comprenant de la lévocétirizine, un système tampon et au moins un excipient hydrosoluble.

17. Composition pharmaceutique selon la revendication 16, la composition permettant une administration orale d'une dose unitaire dans la plage de 1,00 mg à 10,00 mg de lévocétirizine, en tant que substance active, et comprenant
- un noyau qui comprend au moins du mannitol ;
- un premier enrobage appliqué sur le noyau, et comprenant au moins de la bêta-cyclodextrine, le rapport molaire entre la cyclodextrine et la lévocétirizine étant compris entre 0 et 3, et comprenant en outre un système tampon qui contribue à maintenir le pH entre 4,5 et 6,5, lorsque la composition pharmaceutique contenant 5 mg de substance active est dissoute dans 100 ml d'eau ;
- un deuxième enrobage appliqué sur le premier enrobage, et comprenant de la lévocétirizine au moins un excipient hydrosoluble, et un système tampon qui contribue à maintenir le pH entre 4,5 et 6,5, lorsque la composition pharmaceutique contenant 5 mg de substance active est dissoute dans 100 ml d'eau ; et
- facultativement, un enrobage hydrosoluble final appliqué sur le deuxième enrobage.

18. Composition pharmaceutique selon la revendication 11, la composition comprenant
- un noyau qui comprend au moins du mannitol ;
- un premier enrobage appliqué sur le noyau, et comprenant au moins de l'hydroxypropylméthylcellulose ou de l'hydroxypropylcellulose ; et
- un deuxième enrobage appliqué sur le premier enrobage, et comprenant de la lévocétirizine, au moins une cyclodextrine et comprenant en outre un système tampon qui contribue à maintenir le pH entre 4,5 et 6,5, lorsque la composition pharmaceutique contenant 5 mg de substance active est dissoute dans 100 ml d'eau.

19. Composition pharmaceutique selon l'une quelconque des revendications précédentes, la composition étant sous la forme d'un comprimé, un comprimé se désintégrant par voie orale et une capsule.

20. Composition pharmaceutique selon les revendications 1 à 18, la composition étant sous la forme de sirop anhydre ou de granulat, qui peut être rempli dans un sachet ou un dispositif de dosage approprié.
